(19) Europäisches Patentamt
European Patent Office
Office européen des brevets

(11) EP 3 727 318 B1

(12) **EUROPEAN PATENT SPECIFICATION**

(45) Date of publication and mention of the grant of the patent:
**19.01.2022 Bulletin 2022/03**

(21) Application number: **18827056.5**

(22) Date of filing: **19.12.2018**

(51) International Patent Classification (IPC):
***A61K 8/49*** (2006.01) ***A61K 8/55*** (2006.01)
***A61K 8/60*** (2006.01) ***A61Q 5/00*** (2006.01)

(52) Cooperative Patent Classification (CPC):
**A61K 8/606; A61K 8/494; A61K 8/55; A61Q 5/002;**
A61K 2800/81; A61K 2800/884

(86) International application number:
**PCT/EP2018/085886**

(87) International publication number:
**WO 2019/121943 (27.06.2019 Gazette 2019/26)**

(54) **PROCESS FOR TREATING KERATIN MATERIALS USING A FLAVIN DERIVATIVE, A POLYMERIZABLE MOLECULE AND LIGHT RADIATION**

VERFAHREN ZUR BEHANDLUNG VON KERATINMATERIALIEN MITTELS EINES FLAVINDERIVATS, EINES POLYMERISIERBAREN MOLEKÜLS UND LICHTSTRAHLUNG

PROCÉDÉ DE TRAITEMENT DES MATIÈRES KÉRATINIQUES UTILISANT UN DÉRIVÉ DE FLAVINE, UNE MOLÉCULE POLYMÉRISABLE ET UN RAYONNEMENT LUMINEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2017 FR 1762637**

(43) Date of publication of application:
**28.10.2020 Bulletin 2020/44**

(73) Proprietor: **L'OREAL**
**75008 Paris (FR)**

(72) Inventors:
- **GREAVES, Andrew**
  **93601 AULNAY-SOUS-BOIS (FR)**
- **MAGNE, Constance**
  **94152 CHEVILLY LA RUE (FR)**
- **PHILIPPE, Michel**
  **93601 AULNAY-SOUS-BOIS (FR)**
- **DAUBRESSE, Nicolas**
  **93400 SAINT-OUEN (FR)**

(74) Representative: **L'Oreal**
**Service D.I.P.I.**
**9, rue Pierre Dreyfus**
**92110 Clichy (FR)**

(56) References cited:
**WO-A1-2016/126121**
**WO-A2-2011/000648**
**DE-A1-102008 047 945**
**DE-U1- 9 413 358**
**FR-A1- 2 751 218**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 727 318 B1

## Description

The present invention is limited by the scope of the appended claims and relates to a process for treating keratin materials, especially human keratin fibers, comprising the application of a composition comprising i) at least one flavin derivative and ii) at least one polymerizable molecule, preferably a photopolymerizable molecule, and at least one step of exposing said materials to artificial or natural light radiation, in particular for caring for and/or repairing the keratin materials.

The hair is damaged and weakened by external atmospheric agents such as pollution and bad weather, and also by mechanical or chemical treatments, such as brushing, combing, dyeing, bleaching, permanent-waving, relaxing and repeated washing. The hair thus becomes damaged and may in the long run become dry, coarse, brittle, dull, split and/or soft or else sensitive to humidity, making the hair unmanageable, often with frizziness, and/or difficult to style in a humid environment, in particular in a very humid environment. The hair is then also sensitive to mechanical constraints such as being gripped with an elastic band, for example to make a ponytail, and retains the gripping mark even after the elastic band has been taken off.

To overcome these drawbacks, it is common practice to make use of hair compositions for conditioning the hair by giving it cosmetic properties and also good shaping.

However, the conditioning effect obtained via these hair treatments fades out rapidly over time, and in particular does not have satisfactory persistence with respect to shampoo washing.

Furthermore, these hair compositions have little or no effect on controlling the volume of keratin fibers and/or holding the hairstyle and/or managing the hair in a humid or even a very humid environment (humidity-resistant shape and volume control). They also have little or no effect on the sensitivity to mechanical constraints such as being gripped with an elastic band.

There is thus a real need to develop processes for treating keratin fibers that are capable of conserving or even improving the quality of the fiber such as the softness, disentangling, smoothness, manageability, volume and strength of the hair, for example by reducing the brittleness of the hair, and doing so in a persistent manner, or else by making the hair less sensitive to mechanical constraints such as gripping, or by allowing control of the volume and frizziness and/or shape of the keratin fibers and also of the hairstyle in a humid environment, in particular a very humid environment.

In addition, it is known that riboflavin can initiate photochemical reactions such as the crosslinking of polysaccharides to form hydrogels (see, for example, WO 2010/083039, J. Biomed. Mater. Res. B. Appl. Biomater., Kim, S.-H., Chu, C.-C., 91(1), 390-400 (2009). Other hydrogels have been prepared as riboflavin "delivery drug" [see, for example, Drug. Discovery Today, Piyush Gupta, Kavita Vermani and Sanjay Garg, Vol. 7, No. 10 (2002)]. The hydrogels tested with riboflavin or derivatives have not been used in the field of cosmetics.

WO2016/126121 discloses a composition for scalp and hair comprising a yeast extract, wherein the yeast extract may comprise vitamin B2 (riboflavin).

There is still a need to develop a process for treating keratin materials, in particular keratin fibers such as the hair, more particularly a process for treating keratin fibers in an efficient and long-lasting manner while at the same time limiting the perceived degradation of the hair, in particular in a way that is persistent with respect to successive shampoo washes and to control the shape of the humidity-resistant keratin fibers.

There is also a need to develop a process for treating keratin materials, in particular keratin fibers, especially human keratin fibers such as the hair, more particularly a keratin fiber treatment process that is efficient and, if possible, long-lasting while at the same time limiting the perceived degradation of the hair, especially the perception of dry, coarse, brittle, dull, split and/or soft hair or else hair that is sensitive to humidity often with frizziness, and/or that is difficult to style in a humid environment, in particular in a very humid environment, and especially by making the hair insensitive to mechanical constraints such as gripping with an elastic band so as, for example, to make a ponytail.

This (these) aim(s) are achieved by the process of the invention, i.e. a process for treating keratin materials, in particular keratin fibers, especially human keratin fibers such as the hair, comprising:

1) a step of applying a composition (A) comprising:

i) one or more flavin derivatives chosen from the compounds of formula **(I)** below:

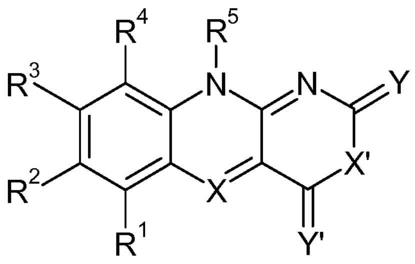

**(I)**

and also the organic or mineral acid or base salts thereof, the optical isomers, geometrical isomers and tautomers thereof, and the solvates thereof such as hydrates; in which formula **(I):**

- **$R^1$**, **$R^2$**, **$R^3$** and **$R^4$**, which may be identical or different, represent a hydrogen atom, a halogen atom or a group chosen from hydroxyl, ($C_1$-$C_5$)alkyl, ($C_1$-$C_6$)alkoxy, ($C_1$-$C_6$)alkylthio, (di)($C_1$-$C_6$)(alkyl)amino, nitro(so), in particular chosen from hydrogen and ($C_1$-$C_5$)alkyl; more particularly, $R^1$ and $R^4$ represent a hydrogen atom and $R^2$ and $R^3$ represent a hydrogen atom or a ($C_1$-$C_4$)alkyl group such as methyl;
- **$R^5$** represents a hydrogen atom or a ($C_1$-$C_8$)alkyl group optionally substituted with one or more groups chosen from i) hydroxyl, ii) $R^6$-C(Y")-O- with $R^6$ representing a hydrogen atom or a ($C_1$-$C_4$)alkyl or aryl($C_1$-$C_4$)alkyl group such as benzyl and Y" represents an oxygen or sulfur atom, or N($R^7$) with $R^7$ representing a hydrogen atom or a ($C_1$-$C_4$)alkyl group, Y" preferably representing an oxygen atom, iii) phosphoric $(HO)_2P(O)$-O-, iv) -O-[(HO)P(O)-O]$_n$-Suc-Het with Suc representing a divalent sugar group such as ribose, and Het representing a heteroaryl group such as adenine, n is an integer equal to 0, 1 or 2, preferably 2; in particular, the group Suc-Het represents a ribose group substituted with an adenine group;
- **X** represents a nitrogen atom or a methylene group C($R^8$) with $R^8$ representing a hydrogen atom or a ($C_1$-$C_4$)alkyl group; preferably, X represents a nitrogen atom;
- **X'** represents an oxygen atom or a group $NR^9$ with $R^9$ representing a hydrogen atom or a ($C_1$-$C_5$)alkyl group; in particular, X' denotes $NR^9$, preferably NH;
- **Y** and **Y'**, which may be identical or different, represent an oxygen or sulfur atom or a group $NR^{10}$ with $R^{10}$ representing a hydrogen atom or a ($C_1$-$C_5$)alkyl group, preferably Y and Y' represent an oxygen atom; and

ii) one or more polymerizable molecules , which are as defined in the appended claims; and

2) a step of supplying energy to said keratin materials, which consists in exposing said materials to at least one artificial or natural light radiation,
it being understood that:

- composition (A) may undergo a step of energy supply before being applied to the keratin materials, said energy supply step consisting in exposing said composition to at least one artificial or natural light radiation to lead to a composition (B) which will then be applied to said materials;
- steps 1) and 2) are possibly performed simultaneously with the application, or sequentially, preferably simultaneously with the application or else sequentially to the application 1) and then 2);
- the polymerizable molecule(s) include at least one insaturated group.

The invention also relates to a process for preparing a composition (B), comprising step a) of mixing i) one or more flavin derivatives chosen from the compounds of formula **(I)** as defined previously, ii) one or more polymerizable molecules as defined below and iii) optionally one or more amines and/or one or more alcohols different i) from the flavins of formula (I), and different ii) from the polymerizable molecules, preferably one or more amines chosen from amino acids; followed by a step b) of exposing said mixture to artificial or natural light radiation.

The invention also relates to a process for preparing a composition (B'), comprising step a) of mixing i) one or more flavin derivatives chosen from the compounds of formula **(I)** as defined previously, ii) one or more polymerizable molecules chosen from those of formula **(II)**, **(IIa)**, **(IIb)** or **(IIc)** as defined below and iii) optionally one or more amines and/or one or more alcohols different i) from the flavins of formula (I), and different ii) from the polymerizable molecules, preferably one or more amines chosen from amino acids; followed by a step b) of exposing said mixture to artificial or natural light radiation.

A subject of the invention is also composition (B) obtained via the process as described previously.

A subject of the invention is also composition (B') obtained via the process as described previously.

A subject of the invention is also a process for treating keratin materials, in particular keratin fibers, especially human keratin fibers such as the hair, comprising a step of applying to said materials a composition (B) or (B') obtained via the preparation process as described previously and optionally a step 2) of supplying energy to said materials, consisting in exposing said materials to at least one artificial or natural light radiation, the optional energy supply step 2) being performed simultaneously with the step of applying composition (B) or (B') or successively to the step of applying composition (B) or (B') to the keratin materials.

A subject of the invention is also a multi-compartment kit or device:

- either comprising in one compartment ingredient i) and in another compartment ingredient ii), or comprising ingredients i) and ii) together in one compartment, and
- in another compartment an artificial-light-emitting device.

Disclosed is also the cosmetic use i) of one or more compounds of formula **(I)** as defined previously in the presence ii) of one or more polymerizable molecules and of at least one artificial or natural light radiation; in particular for caring for and/or repairing keratin materials, especially human keratin fibers such as the hair, preferably for repairing the hair.

The processes of the invention use either a composition (A), (B) or (B') containing at least one flavin derivative of formula (I) as defined previously, especially at least one riboflavin phosphate derivative (vitamin B2 derivative), optionally an amino acid such as arginine and at least one polymerizable and especially photocrosslinkable molecule, followed by a step of irradiating with light radiation, and make it possible to improve the cosmetic qualities of keratin fibers. The fibers appear repaired. Fibers treated via one of the processes of the invention after they have been marked or deformed by a mechanical constraint, such as during the gripping of the fibers with a clip, hair slides, or other elastic means such as an elastic band, for example with a ponytail, are improved relative to untreated fibers where the ponytail leaves a long-lasting imprint on the fibers. With one of the processes of the invention, the imprint disappears rapidly, or even immediately.

Other characteristics and advantages of the invention will emerge more clearly on reading the description and the examples that follow.

In the following text, unless indicated otherwise:

■ the term "*salt*" means the salts of addition with an organic or mineral acid or base;

■ the term "*organic or mineral acid salt*" means cosmetically acceptable addition salts obtained by addition of an organic or mineral acid, more particularly the salts chosen from a salt derived from i) hydrochloric acid HCl, ii) hydrobromic acid HBr, iii) sulfuric acid $H_2SO_4$, iv) alkylsulfonic acids: Alk-S(O)$_2$OH such as methylsulfonic acid and ethylsulfonic acid; v) arylsulfonic acids: Ar-S(O)$_2$OH such as benzenesulfonic acid and toluenesulfonic acid; vi) citric acid; vii) succinic acid; viii) tartaric acid; ix) lactic acid; x) alkoxysulfinic acids: Alk-O-S(O)OH such as methoxysulfinic acid and ethoxysulfinic acid; xi) aryloxysulfinic acids such as tolueneoxysulfinic acid and phenoxysulfinic acid; xii) phosphoric acid $H_3PO_4$; xiii) acetic acid $CH_3C(O)OH$; xiv) triflic acid $CF_3SO_3H$; and xv) tetrafluoroboric acid $HBF_4$;

■ the term "*alkyl*" means a linear or branched radical containing from 1 to 12 carbon atoms, in particular from 1 to 8 carbon atoms, more particularly from 1 to 6 carbon atoms, preferably from 1 to 4 carbon atoms, for example methyl, ethyl, *n*-propyl, isopropyl, butyl, *n*-pentyl, *n*-hexyl, preferably methyl or *n*-propyl and more preferentially methyl;

■ the term "*($C_x$-$C_y$)alkyl*" means an alkyl radical as defined previously, said alkyl radical comprising x to y carbon atoms;

■ the term "*alkylene*" means a linear or branched divalent radical containing from 1 to 16 carbon atoms, in particular from 1 to 12 carbon atoms, preferably from 1 to 8 carbon atoms, more preferentially from 1 to 6 carbon atoms, even more preferentially from 1 to 4 carbon atoms, for example methylene, ethylene, *n*-propylene, isopropylene, butylene, *n*-pentylene or *n*-hexylene, preferably methylene;

■ the term "*($C_x$-$C_y$)alkylene*" means an alkylene radical as defined previously, said alkylene radical comprising x to y carbon atoms;

■ the term "*alkoxy*" means an alkyl-oxy group with "*alkyl*" as defined previously;

■ the term "*($C_x$-$C_y$)alkoxy*" means an alkoxy radical as defined previously, said alkoxy radical comprising x to y carbon atoms;

■ an "*aryl*" radical represents a monocyclic or polycyclic fused or non-fused carbon-based group, comprising from 6 to 22 carbon atoms, at least one ring of which is aromatic; in particular, the aryl radical is a phenyl, biphenyl, naphthyl, indenyl, anthracenyl or tetrahydronaphthyl, preferably phenyl;

■ a "*heteroaryl radical*" represents a 5- to 22-membered, monocyclic or polycyclic, fused or non-fused group, comprising from 1 to 6 heteroatoms chosen from nitrogen, oxygen and sulfur atoms, at least one ring of which is

aromatic; preferentially, a heteroaryl radical is chosen from acridinyl, benzimidazolyl, benzobistriazolyl, benzopyrazolyl, benzopyridazinyl, benzoquinolyl, benzothiazolyl, benzotriazolyl, benzoxazolyl, pyridinyl, pyrimidyl, pyrimidylone, pyrimidyl-dione, tetrazolyl, dihydrothiazolyl, imidazopyridinyl, imidazolyl, indolyl, isoquinolyl, naphthoimidazolyl, naphthoxazolyl, naphthopyrazolyl, oxadiazolyl, oxazolyl, oxazolopyridyl, phenazinyl, phenoxazolyl, pyrazinyl, pyrazolyl, purinyl-one, purinylepyrazoyltriazyl, pyridyl, pyridinoimidazolyl, pyrrolyl, quinolyl, tetrazolyl, thiadiazolyl, thiazolyl, thiazolopyridinyl, thiazoylimidazolyl, thiopyrylyl, triazolyl;

■ the term "*sugar*" or "*Suc*" means a monosaccharide group or a disaccharide group, preferably a monosaccharide, each saccharide unit (the saccharide unit in the case of a monosaccharide or each saccharide unit in the case of a disaccharide) comprising one or more hydroxyl groups optionally substituted with a radical **R"** chosen from: i) $(C_1-C_6)$alkyl, $(C_2-C_6)$alkenyl; ii) an acetyl radical; or iii) a protecting group (PG) for hydroxyl functions, such as $(C_2-C_6)$alkyl(thio)carbonyl, preferably $(C_2-C_6)$alkylcarbonyl; in particular **R"** represents a $(C_1-C_6)$alkyl group such as methyl, or an acetyl group; said monosaccharide or disaccharide radical being linked to the rest of the molecule via a bond between the carbon atom $C_1$ of one of the sugars of said monosaccharide or disaccharide radical, this bond possibly being $\alpha$ or $\beta$ anomeric; it is understood that, for the compounds of formula **(I)** as defined previously, when **Suc** represents a monosaccharide radical, then it is in pyranose form (the sugar heterocycle which constitutes it is 6-membered) or furanose form (the sugar heterocycle which constitutes it is 5-membered); and when **Suc** represents a disaccharide radical, it comprises a sequence of two identical or different saccharide or oside units which may be in furanose or pyranose form. Preferably, the disaccharide results from the sequence of a saccharide unit in furanose form and a unit in pyranose form or the sequence of a saccharide unit in pyranose form and a unit in furanose form; whether it is for the monosaccharide or polysaccharide radical, each saccharide unit may be in levorotatory L or dextrorotatory D form, and in $\alpha$ or $\beta$ anomeric form;

■ the term "*polymerizable molecules*" means any molecule, commonly referred to as a synthon, which includes at least one reactive group that can react with another synthon to form oligomers or polymers; the reactive group being an unsaturated group, wherein the unsaturated group is preferably $-C\equiv CR'$ or $-CR=CR'_2$ with R and R', which may be identical or different, representing a hydrogen atom, a $(C_1-C_4)$alkyl group such as methyl or carboxyl (COOH), preferably a hydrogen atom or a $(C_1-C_4)$alkyl group such as methyl;

■ the term "*polysaccharides*" means a polysaccharide sugar which is a polymer constituted of several saccharides bonded together via O-oside bonds, said polymers being constituted of monosaccharide units as defined previously, said monosaccharide units comprising at least 5 carbon atoms, preferably 6; in particular, the monosaccharide units are linked together via a 1,4 or 1,6 bond as $\alpha$ (alpha) or $\beta$ (beta) anomer, it being possible for each oside unit to be of L or D configuration, and also the salts thereof and the solvates thereof such as the hydrates of said monosaccharides; more particularly, they are polymers formed from a certain number of saccharides (or monosaccharides) having the general formula: $-[C_x(H_2O)_y]_n-$ where x is an integer greater than or equal to 5, preferably x is greater than or equal to 6, in particular x is between 5 and 7 inclusive and preferably x = 6, and y is an integer which represents x - 1, and n is an integer greater than or equal to 2, particularly of between 3 and 3000 inclusive, more particularly between 5 and 2500 and preferentially between 10 and 2300;

■ the term "*amino monosaccharide or polysaccharide*" means that the monosaccharide or polysaccharide is substituted with one or more amino groups $NR_1R_2$, i.e. at least one of the hydroxyl groups of at least one saccharide unit is replaced with a group $NR_1R_2$ with $R_1$ and $R_2$, which may be identical or different, representing i) a hydrogen atom, ii) a $(C_1-C_6)$alkyl group that is optionally substituted, preferably with one or more hydroxyl or $NH_2$ groups, iii) an aryl group such as phenyl, iv) an aryl$(C_1-C_4)$alkyl group such as benzyl, v) a (hetero)cyclo$(C_5-C_7)$alkyl group such as cyclohexyl, morpholinyl, piperazinyl, piperidinyl, vi) a (hetero)cyclo$(C_5-C_7)$alkyl$(C_1-C_4)$alkyl group such as cyclohexylmethyl, vii) $-C(Y)-(Y')_p-R'_1$ with Y and Y', which may be identical or different, representing an oxygen atom, a sulfur atom or $N(R'_2)$, preferably oxygen, p = 0 or 1, preferably 0; and $R'_1$ and $R'_2$ representing i) to vi) of $R_1$ and $R_2$ defined previously, and in particular $R'_1$ denoting a $(C_1-C_5)$alkyl group such as methyl. Preferably $R_1$ and $R_2$ represent a hydrogen atom or a $(C_1-C_4)$alkylcarbonyl group such as acetyl;

■ moreover, the addition salts that may be used in the context of the invention are especially chosen from salts of addition with a cosmetically acceptable base such as the basifying agents as defined below, for instance alkali metal or alkaline-earth metal hydroxides such as sodium hydroxide or potassium hydroxide, ammonia, amines or alkanolamines;

■ the term "*heterocycle*" means a 5- to 10-membered monocyclic or bicyclic, preferably monocyclic, fused or non-fused, saturated or unsaturated, aromatic or non-aromatic group, containing from 1 to 3 heteroatoms chosen from nitrogen, oxygen and sulfur atoms, this heterocycle possibly being substituted with one or more radicals, which may be identical or different, chosen from alkyl, hydroxyalkyl and alkoxy radicals; preferentially, according to the present invention, the heterocycle is saturated or unsaturated, preferably saturated, and 5- to 8-membered, and more preferentially is chosen from piperidyl, pyrrolidinyl, piperazinyl and morpholinyl;

■ the term "*cycloalkyl*" means a 5- to 10-membered monocyclic or bicyclic, preferably monocyclic, fused or non-fused hydrocarbon-based group which is saturated or contains one or more ethylenic unsaturations, which is non-aromatic, this cycloalkyl possibly being substituted with one or more radicals, which may be identical or different, chosen from alkyl, hydroxyalkyl and alkoxy radicals; preferentially, according to the present invention, the cycloalkyl is saturated and 5- to 8-membered, and more preferentially is chosen from cyclopentyl and cyclohexyl;

■ the "*heterocycle*", "*cycloalkyl*", "*aryl*" and "*heteroaryl*" radicals may be substituted with at least one substituent borne by a carbon atom or a heteroatom, chosen from:

- an optionally substituted $C_1$-$C_6$ alkyl radical;
- a halogen atom;
- a hydroxyl group;
- a $C_1$-$C_2$ alkoxy radical;
- a (poly)hydroxy($C_2$-$C_4$)alkoxy radical;
- an amino radical;
- a 5- or 6-membered heterocycloalkyl radical;
- a 5- or 6-membered heteroaryl radical, optionally substituted with a ($C_1$-$C_4$)alkyl radical, preferentially methyl;
- an amino radical substituted with one or two identical or different $C_1$-$C_6$ alkyl radicals, optionally bearing at least:

  i) a hydroxyl group,
  ii) an amino group optionally substituted with one or two optionally substituted $C_1$-$C_3$ alkyl radicals, said alkyl radicals possibly forming with the nitrogen atom to which they are attached a saturated or unsaturated, optionally substituted 5- to 7-membered heterocycle, optionally comprising at least one other nitrogen or non-nitrogen heteroatom,

  - an acylamino radical (-NR-C(O)-R') in which the radical R is a hydrogen atom or a $C_1$-$C_4$ alkyl radical optionally bearing at least one hydroxyl group and the R' radical is a $C_1$-$C_2$ alkyl radical;
  - a carbamoyl radical ($(R)_2N$-C(O)-) in which the radicals R, which may be identical or different, represent a hydrogen atom or a $C_1$-$C_4$ alkyl radical optionally bearing at least one hydroxyl group;
  - an alkylsulfonylamino radical (R'-$S(O)_2$-N(R)-) in which the radical R represents a hydrogen atom or a $C_1$-$C_4$ alkyl radical optionally bearing at least one hydroxyl group and the radical R' represents a $C_1$-$C_4$ alkyl radical or a phenyl radical; an aminosulfonyl radical ($(R)_2N$-$S(O)_2$-) in which the radicals R, which may be identical or different, represent a hydrogen atom or a $C_1$-$C_4$ alkyl radical optionally bearing at least one hydroxyl group;
  - a carboxylic radical in acid or salified (preferably with an alkali metal or a substituted or unsubstituted ammonium) form;
  - a cyano group;
  - a nitro or nitroso group;
  - a polyhaloalkyl group, preferentially trifluoromethyl;

■ the term "*(di)($C_x$-$C_y$)(alkyl)amino*" means an amino radical $NH_2$, a ($C_x$-$C_y$)alkylamino radical, a dialkylamino radical in which each alkyl group is, independently, a $C_x$-$C_y$ alkyl radical;

■ the term "*elastic means*" means any means for gripping keratin fibers, in the form of a short yarn, band or strip comprising at least one part made of elastic material such as rubber, of generally circular shape,

■ the expression *"at least* one" is equivalent to *"one or more";* and

■ the expression *"inclusive"* for a range of concentrations means that the limits of the range are included in the defined interval.

**i) The flavins of formula (I)**

Compositions (A), (B) or (B') of the invention comprise at least one compound of formula **(I)** as defined previously.

According to a particular embodiment of the invention, the compounds of formula **(I)** are such that $\mathbf{R^1}$, $\mathbf{R^2}$, $\mathbf{R^3}$ and $\mathbf{R^4}$, which may be identical or different, represent a hydrogen atom, ($C_1$-$C_6$)alkyl, ($C_1$-$C_6$)alkoxy or (di)($C_1$-$C_4$)(alkyl)amino, in particular chosen from hydrogen and ($C_1$-$C_4$)alkyl. Preferably, $R^1$ and $R^4$ represent a hydrogen atom, and $R^2$ and $R^3$ represent a hydrogen atom or a ($C_1$-$C_4$)alkyl group such as methyl, preferably a ($C_1$-$C_4$)alkyl group such as methyl.

According to a particular embodiment of the invention, the compounds of formula **(I)** are such that $\mathbf{R^5}$ represents a ($C_1$-$C_6$)alkyl group such as methyl.

According to another preferred embodiment of the invention, the compounds of formula **(I)** are such that $\mathbf{R^5}$ represents a ($C_1$-$C_6$)alkyl group substituted with one or more identical or different groups chosen from i) hydroxyl, ii) $R^6$-C(O)-O- with $R^6$ representing a hydrogen atom or a ($C_1$-$C_4$)alkyl group such as methyl or n-propyl; iii) phosphoric

$(HO)_2P(O)$-O-, iv) -O-[(HO)P(O)-O]$_n$-Suc-Het with Suc representing a monosaccharide such as ribose, and Het representing an optionally substituted heteroaryl group, in particular optionally substituted pyrimidyl-dione such as adenine, n is an integer equal to 1 or 2, preferably 2; in particular, Suc-Het represents a ribose group, preferably α-D-ribose, optionally substituted with an adenine group.

According to a preferred embodiment, the compounds of formula **(I)** are such that the radical Suc represents a divalent monosaccharide radical in which the heterocycle constituting it contains 4 or 5 carbon atoms, having the following formula:

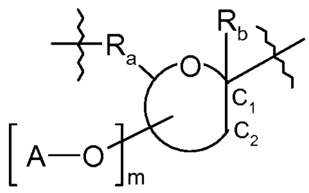

- ■ $\mathbf{R_a}$ representing a $(C_1$-$C_4)$alkylene group such as methylene and $R_a$ being linked to the rest of the molecule via at least one phosphate group, it being understood that the radical $R_a$ is in position $C^5$ if the sugar unit is in pyranose form or in position $C^4$ if it is in furanose form;
- ■ $\mathbf{R_b}$ represents a hydrogen atom or a group $-CH_2$-O-A; preferably, $R_b$ denotes a hydrogen atom;
  **A** represents a hydrogen atom, a $(C_1$-$C_6)$alkyl group or a hydroxy-function-protecting group, such as $R_c$-C(O)- with $R_c$ representing a hydrogen atom or a $(C_1$-$C_4)$alkyl group such as methyl, or else, when n is greater than or equal to 2 and two groups A-O are contiguous, then two groups A may together form a linear or branched $(C_1$-$C_6)$alkylene chain; preferably, all the groups which protect **A** are identical;
- ■ **m** is equal to 1, 2 or 3; preferably, m = 2;
- ■



represents the two substitution parts of the divalent monosaccharide.

According to a more particular embodiment, the compounds of formula **(I)** are such that $\mathbf{R^5}$ represents the following group:

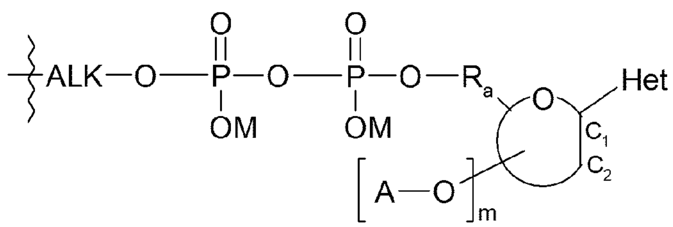

with $\mathbf{R_a}$, **A**, **Het** and **m** as defined previously;

- ALK representing a $(C_1$-$C_6)$alkylene group, preferably pentylene, optionally substituted with one or more hydroxyl groups;
- M represents a hydrogen atom or a cationic counterion, in particular an alkali metal or alkaline-earth metal such as $Na^+$, $K^+$ or ammonium, preferably $Na^+$ and
-

representing the part that is linked to the rest of the molecule.

Preferably, Het represents a purine or pyrimidine nitrogenous base such as adenine, thymine, guanine, cytosine or uracil. Preferably, Het represents an optionally substituted bicyclic heteroaryl group; more particularly, Het represents a purinyl-dione group which is optionally substituted especially with one or more (di)($C_1$-$C_4$)(alkyl)amino groups; preferentially, Het is a purine nitrogenous base such as adenine.

According to a more particular embodiment, the compounds of formula **(I)** are such that $\mathbf{R^5}$ represents a ($C_1$-$C_6$)alkyl group optionally substituted with one or more hydroxyl or phosphate groups such as pentyl substituted with one, two, three or four hydroxyl groups and optionally with a phosphate group, such as 2,3,4,5-tetrahydroxypentyl or 5-dihydrogenophosphate-2,3,4-trihydroxypentyl.

According to a particular embodiment, the compounds of formula **(I)** are such that **X** represents a nitrogen atom.

According to a particular embodiment, the compounds of formula **(I)** are such that **X'** represents a group $NR^9$ with $R^9$ representing a hydrogen atom or a ($C_1$-$C_5$)alkyl group; in particular, X' preferably denotes NH.

According to a particular embodiment, the compounds of formula **(I)** are such that **Y** and **Y'** are identical and more particularly represent an oxygen or sulfur atom; preferably, Y and Y' represent an oxygen atom.

More particularly, the compounds of formula **(I)** of the invention are chosen from compounds 1 to 7 below:

| Structure | |
|---|---|
| 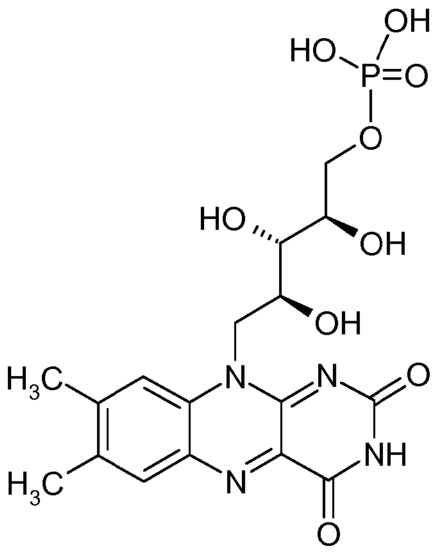 (2R,3S,4S)-5-(7,8-dimethyl-2,4-dioxo-3,4-dihydrobenzo[g]pteridin-10(2H)-yl)-2,3,4-trihydroxypentyl dihydrogen phosphate or riboflavin 5'-phosphate | 1 |
| 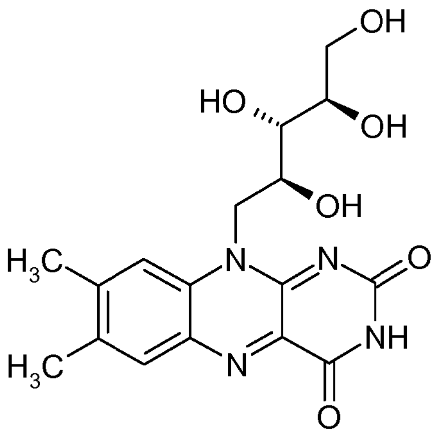 7,8-dimethyl-10-((2*R*,3*R*,4*S*)-2,3,4,5-tetrahydroxypentyl)benzo[g]pteridine-2,4-(3H,10H)-dione, or vitamin $B_2$, lactoflavin | 2 |
| | 3 |

| | |
|---|---|
| 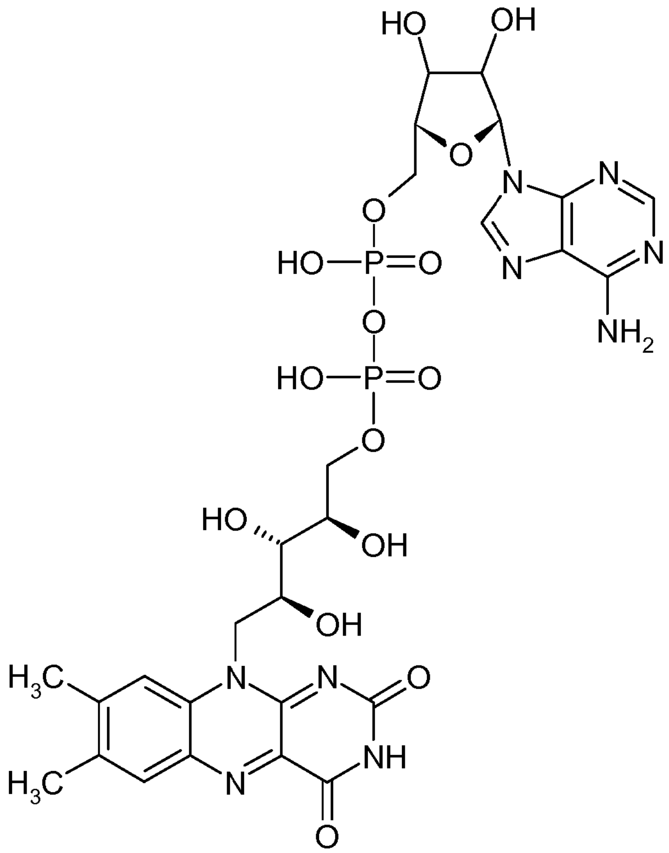<br>Flavin adenine dinucleotide (FAD) | |
| 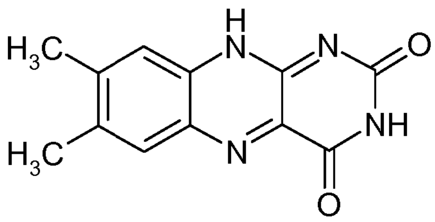<br>7,8-dimethylbenzo[g]pteridine-2,4(3H,10H)-dione | 4 |
| 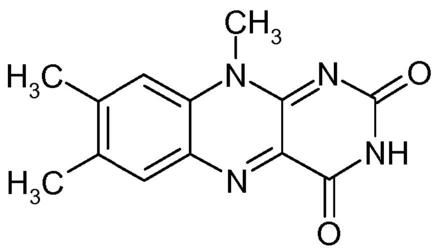<br>7,8,10-trimethylbenzo[g]pteridine-2,4(3H,10H)-dione | 5 |

| | |
|---|---|
| 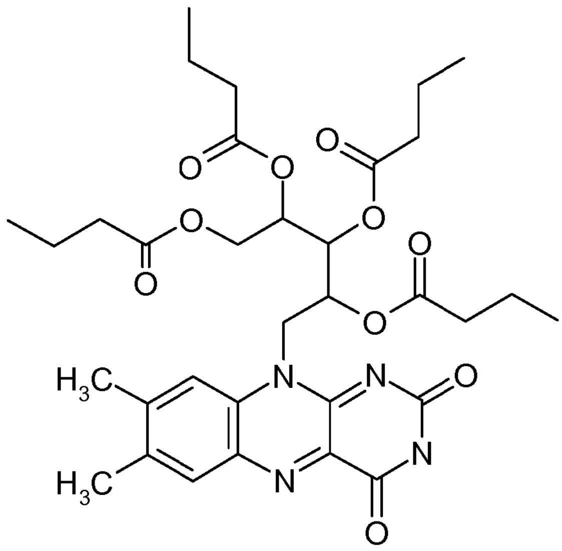<br>Riboflavin tetrabutyrate CAS # 752-56-7 | 6 |
| 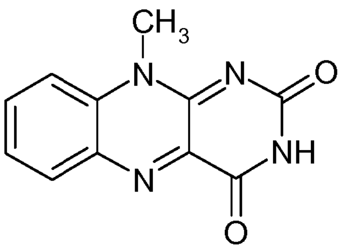<br>10-methylbenzo[g]pteridine-2,4(3H,10H)-dione CAS = 4074-58-2 | 7 |

and also the organic or mineral acid or base salts thereof, the optical isomers and tautomers thereof, and the solvates thereof such as hydrates, in particular the salt thereof with an alkali metal such as sodium.

According to a particular embodiment, compositions (A), (B) or (B') comprise one or more compounds of formula **(I)** as defined previously, in an amount inclusively between 0.01% and 30% by weight relative to the total weight of composition (A), (B) or (B'), in particular between 0.05% and 20%, more particularly between 0.1% and 10%, preferentially between 1% and 5% by weight relative to the total weight of composition (A), (B) or (B').

## ii) The Polymerizable molecules

The disclosed polymerizable molecule(s) have a molecular weight of less than or equal to 500 g/mol, preferably a molecular weight inclusively between 26 and 400 g/mol.

Preferably, the polymerizable molecules are photo-polymerizable, i.e. they have the capacity of polymerizing under exposure to at least one natural and/or artificial light radiation.

Preferably, the polymerizable molecule(s) are chosen from those of formula **(II)** below:

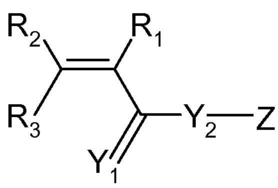

**(II)**

and also the organic or mineral acid or base salts thereof, the optical isomers, geometrical isomers and tautomers thereof, and the solvates thereof such as hydrates; in which formula **(II)**:

- **$R^1$** and **$R^2$**, which may be identical or different, represent a hydrogen atom or a ($C_1$-$C_8$)alkyl or ($C_2$-$C_8$)alkenyl group, said alkyl or alkenyl groups being:

  - optionally interrupted with one or more heteroatoms such as O, S and N($R_5$), with $R^5$ representing a hydrogen atom or a ($C_1$-$C_4$)alkyl group such as methyl, and/or
  - optionally substituted with one or more radicals chosen from the following radicals: hydroxyl, (di)($C_1$-$C_4$)(alkyl)amino, ($C_1$-$C_4$)alkoxy and -C(Y'$_1$)-Y'$_2$-R'$_4$ such as carboxyl;

- **$R_3$** is as defined for $R_2$, or else represents a group -C(Y'$_1$)-Y'$_2$-R'$_4$;
- **$Y_1$**, **$Y_2$**, **Y'$_1$** and **Y'$_2$**, which may be identical or different, represent an oxygen or sulfur atom or N($R_5$), with $R_5$ representing a hydrogen atom or a ($C_1$-$C_4$)alkyl group, preferably, $Y_1$ and Y'$_1$ represent an oxygen atom and $Y_2$ and Y'$_2$ represent an oxygen atom or a group N($R_5$); more particularly, $Y_2$ and Y'$_2$ represent an oxygen atom; and
- **$R_4$** and **R'$_4$**, which may be identical or different, represent a hydrogen atom or a ($C_1$-$C_{16}$)alkyl group optionally substituted with one or more radicals, which may be identical or different, chosen from the following radicals: a) amino ($NH_2$), mono- or di($C_1$-$C_4$)alkylamino such as dimethylamino, b) hydroxyl (OH), c) $SO_3H$, d) carboxyl ($CO_2H$), e) cyano, f) (poly)(halo)($C_1$-$C_4$)alkyl such as trifluoromethyl ($CF_3$), g) ($C_1$-$C_4$)alkoxycarbonyl; said alkyl radical being optionally interrupted with one or more heteroatoms such as O, S and N($R_5$) with $R_5$ as defined previously, and preferably $R_5$ denoting H or Me;
- Z denotes a radical $R_4$ as defined previously or a radical **(Z')** of formula:

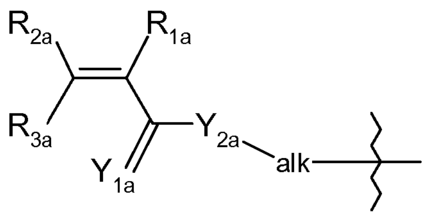

**(Z')**

in which formula **(Z'):**

- $Y_{1a}$, $Y_{2a}$, $R_{1a}$, $R_{2a}$ and $R_{3a}$ have, respectively and independently, the same meaning as $Y_1$, $Y_2$, $R_1$, $R_2$ and $R_3$ defined previously;
- alk denotes a divalent ($C_1$-$C_{16}$)alkylene radical optionally interrupted with one or more oxygen or sulfur atoms or with one or more NH groups, said alkylene radical being optionally substituted with one or more radicals chosen from hydroxyl and a radical of formula (Z"):

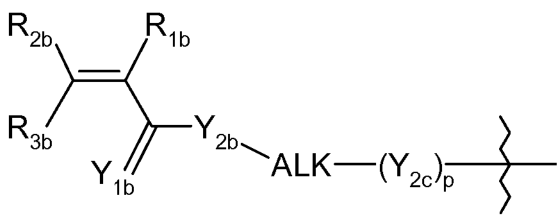

**(Z")**

in which formula **(Z'):**

- $Y_{1b}$, $Y_{2b}$, $Y_{2c}$, $R_{1b}$, $R_{2b}$ and $R_{3b}$ have, respectively and independently, the same meaning as $Y_1$, $Y_2$, $R_1$, $R_2$ and $R_3$ defined previously, ALK denoting a ($C_1$-$C_6$)alkylene radical (divalent radical) such as methylene or propylene and p being equal to 0 or 1;
-

denoting the point of attachment between alk and $Y_2$ in the case of formula (Z') and

-



denoting the point of attachment between $(Y_2)p$ and alk in the case of formula (Z").

According to a particular variant of the invention, **Z** denotes a radical $\mathbf{R_4}$.
According to one embodiment, the polymerizable molecule(s) are of formula **(II)** in which **Z** denotes a radical $\mathbf{R_4}$ and $\mathbf{R_1}$ represents a hydrogen atom or a $(C_1\text{-}C_4)$alkyl group. According to one embodiment, the polymerizable molecule(s) are of formula **(II)** in which Z denotes a radical $\mathbf{R_4}$ and $\mathbf{R_1}$ represents a hydrogen atom. According to another particular embodiment, the polymerizable molecule(s) are of formula **(II)** in which **Z** denotes a radical $\mathbf{R_4}$ and $\mathbf{R_1}$ represents a $(C_1\text{-}C_4)$alkyl group such as methyl.
According to another embodiment, the polymerizable molecule(s) are of formula **(II)** in which **Z** denotes a radical $\mathbf{R_4}$ and $\mathbf{R_1}$ represents a $(C_1\text{-}C_4)$alkyl group substituted with a radical chosen from hydroxyl and $-C(Y'_1)\text{-}Y'_2\text{-}R'_4$, in particular $-C(O)\text{-}O\text{-}R'_4$ with $R'_4$ as defined previously; preferably $\mathbf{R_1}$ denotes a $(C_1\text{-}C_4)$alkyl group substituted with a carboxyl group such as carboxymethyl.
According to one embodiment, the polymerizable molecule(s) are of formula **(II)** in which **Z** denotes a radical $\mathbf{R_4}$ and $\mathbf{R_2}$ represents a hydrogen atom, a $(C_1\text{-}C_4)$alkyl group optionally substituted with one or more radicals chosen from hydroxyl, (di)$(C_1\text{-}C_4)$(alkyl)amino, $(C_1\text{-}C_4)$alkoxy and carboxyl radicals, $R_2$ preferably represents a hydrogen atom or a $(C_1\text{-}C_4)$alkyl group such as methyl, in particular a hydrogen atom.
According to one embodiment, the polymerizable molecule(s) are of formula **(II)** in which Z denotes a radical $\mathbf{R_4}$ and $\mathbf{R_3}$ represents i) a hydrogen atom, ii) a $(C_1\text{-}C_4)$alkyl group optionally substituted with one or more radicals chosen from hydroxyl, (di)$(C_1\text{-}C_4)$(alkyl)amino, $(C_1\text{-}C_4)$alkoxy and carboxyl radicals; iii) $-C(O)\text{-}O\text{-}R'_4$.
According to one embodiment, the polymerizable molecule(s) are of formula **(II)** in which Z denotes a radical $\mathbf{R_4}$ and $\mathbf{R_3}$ represents i) a hydrogen atom.
According to another embodiment, the polymerizable molecule(s) are of formula **(II)** in which Z denotes a radical $\mathbf{R_4}$ and $\mathbf{R_3}$ represents a $(C_1\text{-}C_4)$alkyl group optionally substituted with a radical chosen from hydroxyl and carboxyl.
According to another embodiment, the polymerizable molecule(s) are of formula **(II)** in which Z denotes a radical $\mathbf{R_4}$ and $\mathbf{R_3}$ represents $-C(O)\text{-}O\text{-}R'_4$ with $R'_4$ as defined previously.
According to one embodiment, the polymerizable molecule(s) are of formula **(II)** in which Z denotes a radical $\mathbf{R_4}$ and $\mathbf{R_4}$ and $\mathbf{R'_4}$, which may be identical or different, represent i) a hydrogen atom, or ii) a $(C_1\text{-}C_4)$alkyl group such as methyl, preferably a hydrogen atom.
According to another embodiment, the polymerizable molecule(s) are of formula (II) in which Z denotes a radical $\mathbf{R_4}$, and $\mathbf{R_4}$ and $\mathbf{R'_4}$, which may be identical or different, represent a $(C_1\text{-}C_{16})$alkyl group substituted with one or more radicals, which may be identical or different, chosen from the following radicals: amino ($NH_2$), mono- or di$(C_1\text{-}C_4)$alkylamino such as dimethylamino, hydroxyl (OH), $SO_3H$, carboxyl ($CO_2H$), cyano, trifluoromethyl ($CF_3$), $(C_1\text{-}C_4)$alkyloxycarbonyl, which is preferably monosubstituted; according to this embodiment, $\mathbf{R_4}$ and $\mathbf{R'_4}$, which may be identical or different, in particular represent a $(C_1\text{-}C_6)$alkyl group substituted with one or more radicals, which may be identical or different, chosen from the following radicals: amino ($NH_2$), mono- or di$(C_1\text{-}C_4)$alkylamino such as dimethylamino, hydroxyl (OH), $SO_3H$, carboxyl ($CO_2H$), cyano, said radical preferably being monosubstituted, such as the 2-hydroxypropyl radical.
According to another embodiment, the polymerizable molecule(s) are of formula **(II)** in which **Z** denotes a radical $\mathbf{R_4}$, and $\mathbf{R_4}$ and $\mathbf{R'_4}$, which may be identical or different, represent a $(C_1\text{-}C_{15})$alkyl group interrupted with one or more heteroatoms, such as O, S and $N(R_5)$ with $R_5$ as defined previously, and preferably $R_5$ denoting H or Me; according to this embodiment, $\mathbf{R_4}$ and $\mathbf{R'_4}$, which may be identical or different, represent a $(C_1\text{-}C_{16})$alkyl group interrupted with one or more non-adjacent oxygen atoms and especially a radical $-(CH_2CH_2O)_n\text{-}R$ in which n denotes an integer equal to 0 or 1 or 2 or 3 or 4 or 5 and R denotes a $C_1\text{-}C_6$ alkyl radical such as methyl or ethyl, or alternatively $\mathbf{R_4}$ and $\mathbf{R'_4}$ independently denote a 2-ethoxyethyl radical.
According to a particular variant of the invention, the polymerizable molecule(s) are of formula **(II)** in which **Z** denotes a radical of formula **(Z')**.
Preferably, the polymerizable molecules of formula (II) are such that **Z** denotes a radical of formula **(Z')** and **(Z')** represents a group chosen from:

a) $-(CH_2)_nX$ with X representing a group from among: hydroxyl, amino, (di)(C1-C4)alkylamino such as dimethylamino, sulfonic $-SO_3H$, carboxyl or cyano and n representing an integer between 1 and 5 inclusive;
b) $-(CH_2CH_2O)_n\text{-}R$ and n is as defined above, R representing a hydrogen atom or a $(C_1\text{-}C_4)$alkyl group such as methyl;

c) $-(CH_2)_n(CF_3)_m$ and n is equal to 1, 2 or 3 and m is an integer between 1 and 8 inclusive.

According to one embodiment, the polymerizable molecule(s) are of formula **(II)** in which **Z** denotes a radical of formula **(Z')**, and $\mathbf{Y_{1a}}$ and $\mathbf{Y_{2a}}$ represent an oxygen atom.

According to one embodiment, the polymerizable molecule(s) are of formula **(II)** in which **Z** denotes a radical of formula **(Z')** and $\mathbf{R_1}$ represents a hydrogen atom or a $(C_1-C_4)$alkyl group such as methyl.

According to one embodiment, the polymerizable molecule(s) are of formula **(II)** in which **Z** denotes a radical of formula **(Z')** and $\mathbf{R_1}$ represents a hydrogen atom.

According to another embodiment, the polymerizable molecule(s) are of formula **(II)** in which **Z** denotes a radical of formula **(Z')** and $\mathbf{R_1}$ represents a $(C_1-C_4)$alkyl group substituted with a radical chosen from hydroxyl and $-C(Y'_1)-Y'_2-R'_4$, in particular $-C(O)-OR'_4$ with $R'_4$ as defined previously; preferably $\mathbf{R_1}$ denotes a $(C_1-C_4)$alkyl group substituted with a carboxyl group such as carboxymethyl.

According to one embodiment, the polymerizable molecule(s) are of formula **(II)** in which **Z** denotes a radical of formula **(Z')** and $\mathbf{R_2}$ represents a hydrogen atom or a $(C_1-C_4)$alkyl group optionally substituted with one or more radicals chosen from hydroxyl, (di)$(C_1-C_4)$(alkyl)amino, $(C_1-C_4)$alkoxy and carboxyl radicals; preferably, $\mathbf{R_2}$ represents a hydrogen atom.

According to one embodiment, the polymerizable molecule(s) are of formula **(II)** in which **Z** denotes a radical of formula **(Z')** and $\mathbf{R_3}$ represents i) a hydrogen atom, ii) a $(C_1-C_4)$alkyl group optionally substituted with one or more radicals chosen from hydroxyl, (di)$(C_1-C_4)$(alkyl)amino, $(C_1-C_4)$alkoxy and carboxyl radicals; iii) $-C(O)-O-R'_4$.

According to one embodiment, the polymerizable molecule(s) are of formula **(II)** in which **Z** denotes a radical of formula **(Z')** and $\mathbf{R_3}$ represents i) a hydrogen atom. According to another embodiment, the polymerizable molecule(s) are of formula **(II)** in which **Z** denotes a radical of formula **(Z')** and $\mathbf{R_3}$ represents a $(C_1-C_4)$alkyl group optionally substituted with a radical chosen from hydroxyl and carboxyl.

According to another embodiment, the polymerizable molecule(s) are of formula **(II)** in which **Z** denotes a radical of formula **(Z')** and $\mathbf{R_3}$ represents $-C(O)-O-R'_4$ with $R'_4$ as defined previously.

According to one embodiment, the polymerizable molecule(s) are of formula **(II)** in which Z denotes a radical of formula **(Z')**, and $\mathbf{R_4}$ and $\mathbf{R'_4}$, which may be identical or different, represent i) a hydrogen atom, or ii) a $(C_1-C_4)$alkyl group such as methyl, preferably a hydrogen atom.

According to another embodiment, the polymerizable molecule(s) are of formula **(II)** in which **Z** denotes a radical of formula **(Z')**, and $\mathbf{R_4}$ and $\mathbf{R'_4}$, which may be identical or different, represent a $(C_1-C_{16})$alkyl group substituted with one or more radicals, which may be identical or different, chosen from the following radicals: amino $(NH_2)$, mono- or di$(C_1-C_4)$alkylamino such as dimethylamino, hydroxyl (OH), $SO_3H$, carboxyl $(CO_2H)$, cyano, trifluoromethyl $(CF_3)$, $(C_1-C_4)$alkyloxycarbonyl, which is preferably monosubstituted; according to this embodiment, $\mathbf{R_4}$ and $\mathbf{R'_4}$, which may be identical or different, in particular represent a $(C_1-C_5)$alkyl group substituted with one or more radicals, which may be identical or different, chosen from the following radicals: amino $(NH_2)$, mono- or di$(C_1-C_4)$alkylamino such as dimethylamino, hydroxyl (OH), $SO_3H$, carboxyl $(CO_2H)$, cyano, said radical preferably being monosubstituted, such as the 2-hydroxypropyl radical.

According to another embodiment, the polymerizable molecule(s) are of formula **(II)** in which **Z** denotes a radical of formula **(Z')**, and $\mathbf{R_4}$ and $\mathbf{R'_4}$, which may be identical or different, represent a $(C_1-C_{16})$alkyl group interrupted with one or more heteroatoms, such as O, S and $N(R_5)$ with $\mathbf{R_5}$ as defined previously, and preferably $R_5$ denoting H or Me; according to this embodiment, $\mathbf{R_4}$ and $\mathbf{R'_4}$, which may be identical or different, represent a $(C_1-C_{16})$alkyl group interrupted with one or more non-adjacent oxygen atoms and especially a radical $-(CH_2CH_2O)_n-R$ in which n denotes an integer equal to 0 or 1 or 2 or 3 or 4 or 5 and R denotes a $C_1-C_6$ alkyl radical such as methyl or ethyl, or alternatively $\mathbf{R_4}$ and $\mathbf{R'_4}$ independently denote a 2-ethoxyethyl radical.

According to another embodiment, the polymerizable molecule(s) are of formula **(II)** in which **Z** denotes a radical of formula **(Z')** and **alk** denotes a $(C_1-C_{16})$alkylene radical (divalent radical) optionally interrupted with one or more oxygen or sulfur atoms or with one or more NH groups, said divalent radical **alk** preferably denoting a $(C_1-C_{16})$alkylene radical (divalent radical) optionally interrupted with one or more oxygen atoms, such as a radical $-(CH_2-CH_2-O)_q$ with q = 1 to 8.

According to another embodiment, the polymerizable molecule(s) are of formula **(II)** in which **Z** denotes a radical of formula **(Z')** and **alk** denotes a $(C_1-C_{16})$alkylene and preferably $(C_1-C_6)$alkylene radical (divalent radical) such as a $C_5$ alkyl radical or a $C_4$ alkyl radical or a $C_3$ alkyl radical, said radical **alk** being optionally substituted with one or more hydroxyl radical and/or one or more radicals of formula **(Z")** as defined previously, preferably optionally substituted with a hydroxyl radical and substituted with one or two radicals of formula **(Z")** as defined previously. According to this embodiment, when **alk** is substituted with one or two radicals of formula **(Z"):**

- when p = 1, $\mathbf{Y_{2c}}$ preferably denotes an oxygen atom;
- **ALK** preferably denotes a $C_1-C_4$ radical such as methylene or propylene;

- $\mathbf{Y_{1b}}$ and $\mathbf{Y_{2b}}$ are preferably identical and in particular denote oxygen;
- $\mathbf{R_{1b}}$, $\mathbf{R_{2b}}$ and $\mathbf{R_{3B}}$ are preferably identical and in particular denote hydrogen.

More particularly, the polymerizable molecule(s) are chosen from those of formula **(IIa)** below:

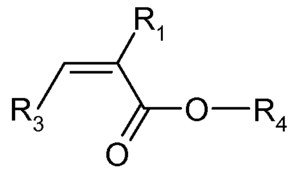

**(IIa)**

and also the organic or mineral acid or base salts thereof, the optical isomers, geometrical isomers and tautomers thereof, and the solvates thereof such as hydrates; in which formula **(IIa)**, $\mathbf{R_1}$, $\mathbf{R_3}$ and $\mathbf{R_4}$ are as defined previously.

In another particular variant, the polymerizable molecule(s) are chosen from those of formula **(IIb)** below:

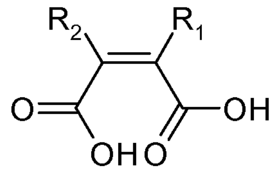

**(IIb)**

and also the organic or mineral acid or base salts thereof, the optical isomers, geometrical isomers and tautomers thereof, and the solvates thereof such as hydrates;
in which formula **(IIb)** $\mathbf{R_1}$ and $\mathbf{R_2}$ are as defined previously, in particular chosen from hydrogen or $(C_1\text{-}C_4)$alkyl such as methyl, preferably hydrogen.

According to another variant, the polymerizable molecules are chosen from those of formula **(IIc)** below:

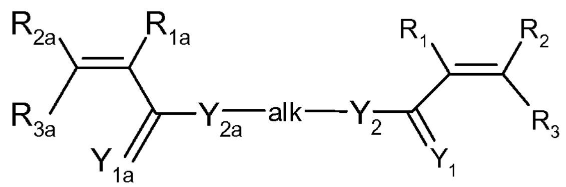

**(IIc)**

and also the organic or mineral acid or base salts thereof, the optical isomers, geometrical isomers and tautomers thereof, and the solvates thereof such as hydrates; in which formula **(IIc)** $\mathbf{R_1}$, $\mathbf{R_2}$, $\mathbf{R_3}$, $\mathbf{Y_1}$, $\mathbf{Y_2}$, **alk**, $\mathbf{Y_{2a}}$, $\mathbf{Y_{1a}}$, $\mathbf{R_{1a}}$, $\mathbf{R_{2a}}$ and $\mathbf{R_{3a}}$ are as defined previously, in particular such that $R_1 = R_{1a}$ and $R_2 = R_{2a}$ and $R_3 = R_{3a}$ and $Y_1 = Y_{1a}$ and $Y_2 = Y_{2a}$.
In particular, the polymerizable molecules are chosen from those of formula **(IIc)** for which:

- $\mathbf{R_1}$ and $\mathbf{R_{1a}}$ are identical and denote hydrogen or $(C_1\text{-}C_4)$alkyl such as methyl, preferably hydrogen;
- $\mathbf{Y_2}$ and $\mathbf{Y_{2a}}$ are identical and denote an oxygen atom or $NR_5$ as defined previously, and in particular $\mathbf{Y_2}$ and $\mathbf{Y_{2a}}$ are identical and denote oxygen or NH;
- $\mathbf{Y_1}$ and $\mathbf{Y_{1a}}$ are identical and denote an oxygen atom;
- **alk** denotes a $(C_1\text{-}C_{16})$alkylene radical (divalent radical) optionally interrupted with one or more oxygen atoms and/or optionally substituted with one or more radicals chosen from hydroxyl and/or a radical of formula **(Z")** as defined previously, said radical(s) **(Z")**, when they are present, preferably being such that $\mathbf{R_{1b}}$ and $\mathbf{R_{2b}}$ and $\mathbf{R_{3b}}$ denote hydrogen, $\mathbf{Y_{1b}}$ and $\mathbf{Y_{2b}}$ denote oxygen, **ALK** denotes a $(C_1\text{-}C_4)$alkylene radical (divalent radical) such as methylene or propylene and **p** is equal to 0 or 1; $\mathbf{Y_{2c}}$, when it is present, denotes an oxygen atom.

According to this variant, the polymerizable molecules are chosen from compounds (a) to (d) below:

| | |
|---|---|
| 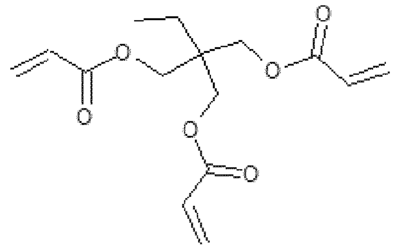 | 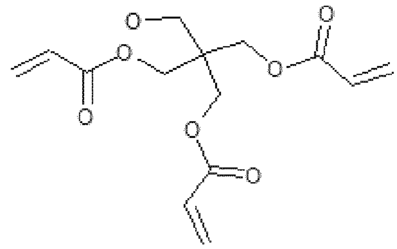 |
| **(a)** | **(b)** |
| 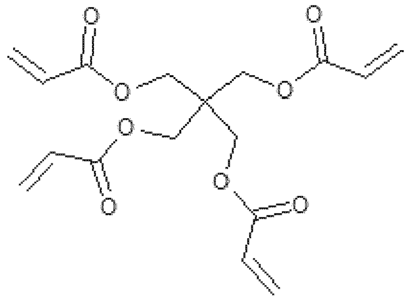 | 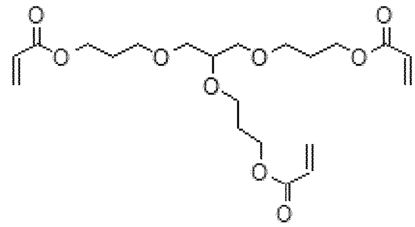 |
| **(c)** | **(d)** |

Preferably, the polymerizable molecule(s) are chosen from (($C_1$-$C_4$)alkyl)acrylic acid such as methacrylic acid, acrylic acid and itaconic acid, in particular itaconic acid and also the organic or mineral base salts thereof and the solvates thereof such as hydrates.

According to a particular embodiment, compositions (A), (B) or (B') comprise one or more polymerizable molecules as defined previously, in an amount inclusively between 0.01% and 50% by weight relative to the total weight of compositions (A), (B) or (B'), in particular between 0.1% and 40%, more particularly between 1% and 30%, preferentially between 2% and 20%, more preferentially between 5% and 10% by weight relative to the total weight of compositions (A), (B) or (B').

**iii) The amines**

Compositions (A), (B) or (B') optionally comprise one or more amines.

According to one variant, compositions (A), (B) or (B') comprise one or more amines.

According to the invention, the term "*amines*" means any organic molecule comprising at least one primary, secondary or tertiary amine group, other than i) the riboflavins of formula (I), and other than ii) the polymerizable molecules. The amines may be aromatic or nonaromatic, saturated or unsaturated, oligomeric or polymeric, and natural or synthetic.

According to a preferred embodiment, the amine(s) of the invention are present in compositions (A), (B) or (B') in an amount of between 0.01% and 15% by weight relative to the total weight of the composition which comprises same, more particularly between 0.05% and 5%, more preferentially between 0.1% and 3% by weight relative to the total weight of the composition which comprises same.

According to a particular embodiment of the invention, compositions (A), (B) or (B') of the invention comprise at least one amino acid, preferably a natural amino acid.

Preferably, the amino acid(s) are chosen from the compounds of formula **(III)** below:

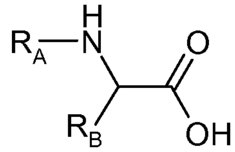

**(III)**

and also the organic or mineral acid or base salts thereof, the optical isomers, geometrical isomers and tautomers

thereof, and the solvates thereof such as hydrates;

- **$R_A$** represents a hydrogen atom, a $(C_1-C_4)$alkyl or $(C_1-C_4)$alkylcarbonyl group or else forms, together with the radical $R_B$ and the nitrogen and carbon atom that bear them, respectively, a 5- to 10-membered monocyclic or bicyclic heterocycle such as pyrrolidinyl; preferably, $R_A$ represents a hydrogen atom;
- **$R_B$** represents a hydrogen atom or a $(C_1-C_5)$alkyl group optionally substituted with one or more groups chosen from i) -Z-C(Z')-Z"-$R_C$ with Z, Z', and Z", which may be identical or different, representing an oxygen or sulfur atom, and N($R_D$), $R_C$ and $R_D$, which may be identical or different, representing a hydrogen atom or a $(C_1-C_4)$alkyl group; preferably, Z, Z' and Z" represent a group N($R_D$) such as NH; ii) (hetero)aryl such as imidazolyl, indolyl, phenyl, optionally substituted especially with a hydroxyl group; iii) (di)$(C_1-C_4)$(alkyl)amino, iv) -C(Z')-Z"-Rc, with Z', Z" and $R_C$ as defined above, in particular Z' represents an oxygen atom, Z" represents an oxygen atom or a group N($R_D$) such as NH, and $R_C$ represents a hydrogen atom; v) -Z‴-$R_C$, with Z'" representing an oxygen, sulfur or selenium atom or an NH group and $R_C$ is as defined previously; in particular, RB represents a $(C_1-C_6)$alkyl group optionally substituted with i) -Z-C(Z')-Z"-Rc, with Z, Z', and Z", which may be identical or different, representing an oxygen or sulfur atom, and N($R_D$), $R_C$ and $R_D$, which may be identical or different, representing a hydrogen atom or a $(C_1-C_4)$alkyl group; preferably Z, Z' and Z" represent a group N($R_D$) such as NH;

or $R_A$ and $R_B$ form, together with the nitrogen atom which bears $R_A$ and with the carbon atom which bears $R_B$, a saturated 5- or 6-membered, preferably 5-membered, heterocycle such as a pyrrolidine ring.

Said amino acids of formula (III) may be of L (levorotatory) or D (dextrorotatory) configuration or in a mixture, preferably of L configuration.

In particular, the amino acid(s) are chosen from alanine, arginine and proline, preferably L-alanine, L-arginine and L-proline, in particular L-arginine or L-proline, more preferentially L-arginine.

According to a preferred embodiment, the amino acid(s) of formula (III) of the invention are present in composition (A) or in composition (B) in an amount of between 0.01% and 15% by weight relative to the total weight of the composition which comprises same, more particularly between 0.05% and 5%, more preferentially between 0.1% and 3% by weight relative to the total weight of the composition which comprises same.

According to another particular embodiment of the invention, composition (A) or (B) of the invention comprises at least one amine comprising an optionally substituted (hetero)aryl group, in particular of formula **(IV):**

$$CYC\text{-}(ALK)_p\text{-}NR_eR_f$$

and also the organic or mineral acid or base salts thereof, the optical isomers thereof, and the solvates thereof such as hydrates;

in which formula **(IV):**

- **CYC** represents an optionally substituted heterocycle, cycloalkyl, aryl or heteroaryl group, preferably aryl such as phenyl,
- **ALK** represents a $(C_1-C_5)$alkylene group,
- **$R_e$** and **$R_f$**, which may be identical or different, represent a hydrogen atom or a $(C_1-C_6)$alkyl group; and
- **p** is equal to 0 or 1.

Preferably, the compounds of formula (IV) are a benzylamine.

According to a preferred embodiment, the amine(s) of formula (IV) are present in composition (A) or in composition (B) in an amount of between 0.01% and 20% by weight relative to the total weight of the composition which comprises same, more particularly between 0.05% and 10%, more preferentially between 0.05% and 5% by weight relative to the total weight of the composition which comprises same.

According to yet another particular embodiment of the invention, composition (A), (B) or (B') comprises at least one amino monosaccharide or at least one amino polysaccharide.

More preferentially, the monosaccharide(s) bearing amine group(s) a) of the invention are hexosamines of formula **(V)** and also the salts thereof with an organic or mineral acid such as hydrochloric acid, and also the α or β anomers thereof, the optical isomers thereof of L or D configuration, and the solvates thereof such as hydrates:

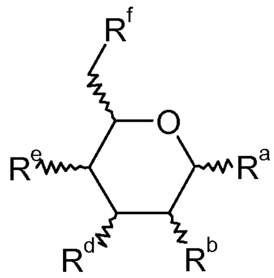

**(V)**

in which formula **(V):**

- **$R^a$**, **$R^b$, $R^d$**, **$R^e$** and **$R^f$**, which may be identical or different, represent i) a hydroxyl group, ii) a $(C_1-C_4)$alkoxy group, the alkyl group of which may be optionally substituted, especially with one or more hydroxyl groups, iii) a carboxyl group, and iv) a group $NR_1R_2$, with $R_1$ and $R_2$, which may be identical or different, chosen from a hydrogen atom, $(C_1-C_4)$alkyl and -C(O)-R'$_1$ with R'$_1$ being as defined previously; preferably, $R_1$ and $R_2$ represent i) a hydrogen atom or ii) an alkylcarbonyl group -C(O)-R'$_1$ with R'$_1$ representing a $(C_1-C_4)$alkyl group such as methyl;

  it being understood that at least one of the radicals **$R^a$**, **$R^b$, $R^d$**, **$R^e$** and **$R^f$** represents a group $NR_1R_2$; preferably at least one of the radicals **$R^a$**, **$R^b$**, **$R^d$**, **$R^e$** and **$R^f$** represents a group $NR_1R_2$ and the radicals different from $NR_1R_2$ denote a hydroxyl group; more particularly, **$R^b$** represents a group $NR_1R_2$, preferentially $NH_2$, and **$R^a$**, **$R^d$**, **$R^e$** and **$R^f$** represent a hydroxyl group. Preferably, the compounds of formula **(V)** are of D configuration, also referred to as D-glucopyrans. In particular, the compounds of formula **(V)** are of β (beta) anomeric configuration. According to a particular mode, the monosaccharide(s) bearing amine group(s) a) of the invention are chosen from the compounds of formula **(V')** below and also the salts thereof with an organic or mineral acid, preferably a mineral acid such as hydrochloric acid, and also the α or β anomers thereof, the optical isomers thereof of L or D configuration, preferably D configuration, and the solvates thereof such as hydrates:

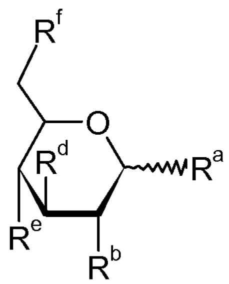

**(V')**

in which formula **(V') $R^a$**, **$R^b$, $R^d$**, **$R^e$** and **$R^f$** are as defined for **(V)** above.

According to a preferred embodiment, the amino monosaccharide(s) of the invention are present in compositions (A), (B) or (B') in an amount of between 0.01% and 20% by weight relative to the total weight of the composition which comprises same, more particularly between 0.05% and 10%, more preferentially between 0.1% and 5% by weight relative to the total weight of the composition which comprises same.

According to yet another particular embodiment of the invention, composition (A), (B) or (B') comprises at least one polysaccharide bearing an amino group, and also the organic or mineral acid salts thereof, the α or β anomers thereof, the optical isomers thereof of L or D configuration and the solvates thereof such as hydrates.

According to a preferred embodiment of the invention, the amino polysaccharide(s) have an average molecular weight MW of less than or equal to 400 kDa, particularly less than 200 kDa.

According to a particular embodiment of the invention, the amino polysaccharide(s) are chosen from those bearing $C_5-C_7$ saccharide units and also the organic or mineral acid salts thereof, the α or β anomers thereof, the optical isomers thereof of L or D configuration, and the solvates thereof such as hydrates.

According to one particular embodiment, the saccharide units of the amino polysaccharide(s) are linked together

between the C1 carbon atoms of one saccharide unit and the C4 carbon atoms of the other saccharide unit, denoted (1→4), such as the amino polysaccharides of formula **(VI)** below, and also the organic or mineral acid salts thereof, the α or β anomers thereof, the optical isomers thereof of L or D configuration, and the solvates thereof such as hydrates:

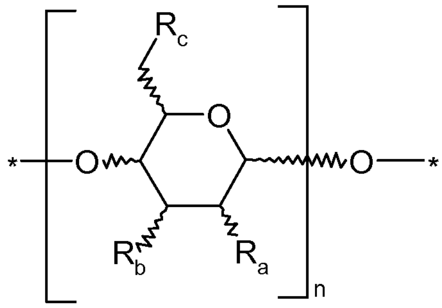

**(VI)**

in which formula **(VI):**

- the $\mathbf{R_a}$, $\mathbf{R_b}$, $\mathbf{R_c}$ radicals of each saccharide unit may be identical or different;
- **n** is an integer greater than or equal to 2, particularly between 3 and 3000 inclusive, and more particularly between 5 and 2500, preferentially between 10 and 2300;
- $\mathbf{R_a}$, $\mathbf{R_b}$, and $\mathbf{R_c}$, which may be identical or different, represent i) a hydroxyl group, ii) a $(C_1-C_4)$alkoxy group, the alkyl group of which may be optionally substituted, especially with one or more hydroxyl groups, iii) a carboxyl group, and iv) a group $NR_1R_2$, with $R_1$ and $R_2$ as defined above, in particular $R_1$ and $R_2$ are chosen from a hydrogen atom and -C(O)-$R'_1$ in which $R'_1$ is as defined above; preferably $R_1$ and $R_2$ represent i) a hydrogen atom or ii) -C(O)-$R'_1$ with $R'_1$ representing a $(C_1-C_4)$alkyl group such as methyl; it being understood that at least one of the $\mathbf{R_a}$, $\mathbf{R_b}$ or $\mathbf{R_c}$ radicals of at least one saccharide unit represents a group $NR_1R_2$ and that at least one of the groups $NR_1R_2$ of at least one saccharide unit represents an $NH_2$ group; preferably, $\mathbf{R_a}$ of at least one saccharide unit represents a group $NR_1R_2$ with $R_1$ which represents a hydrogen atom and $R_2$ is chosen from i) a hydrogen atom or ii) a group -C(O)-$R'_1$, and $\mathbf{R_b}$ and $\mathbf{R_c}$ represent a hydroxyl group, it being understood that at least one of the groups $NR_1R_2$ of at least one saccharide unit represents an $NH_2$ group.

More particularly, the amino polysaccharide(s) of the invention are of formula $\mathbf{(VI_1)}$ below, and also the organic or mineral acid salts thereof, the α or β anomers thereof, the optical isomers thereof of L or D configuration, and the solvates thereof such as hydrates:

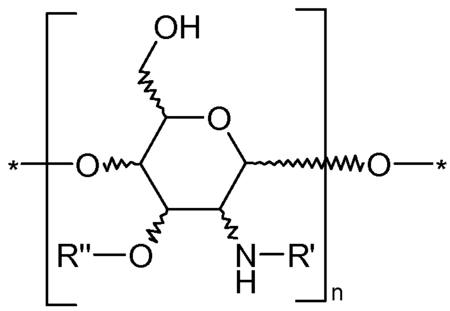

$\mathbf{(VI_1)}$

in which formula $\mathbf{(VI_1)}$:

- **R'** represents a hydrogen atom or a $(C_1-C_4)$alkylcarbonyl group such as acetyl $CH_3$-C(O)-.,
- **R"** represents a hydrogen atom or a $(C_1-C_4)$alkyl group optionally substituted with a carboxyl group such as -CH($CO_2H$)-$CH_3$;
- **n** is an integer greater than or equal to 2, particularly between 3 and 3000 inclusive, more particularly between 5

and 2500, preferentially between 10 and 2300;

it being understood that in the polysaccharide **($VI_1$)** at least one saccharide unit bears an $NH_2$ amino group and at least one other saccharide unit bears at least one N(H)-R' group with R' representing a ($C_1$-$C_4$)alkylcarbonyl group such as acetyl $CH_3$-C(O)-.

Preferentially, the amino polysaccharide(s) of the invention are chosen from chitin and chitosan and derivatives thereof, preferably chitosan.

More particularly, the amino polysaccharide(s) of the invention are chosen from those of formula **($VI_2$)** below, and also the organic or mineral acid salts thereof, and the solvates thereof such as hydrates:

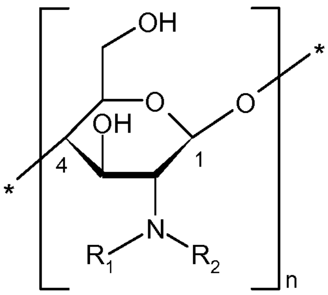

**($VI_2$)**

in which formula **($VI_2$)**:

- **$R_1$** and **$R_2$** are as defined in formula (VI) or ($VI_1$); and
- **n** is an integer greater than or equal to 2, particularly between 3 and 3000 inclusive, more particularly between 5 and 2500, preferentially between 10 and 2300;

it being understood that in the polysaccharide of formula **($VI_2$)** at least one saccharide unit bears an amino group $NH_2$ and at least one other saccharide unit bears a group N(H)-R' with R' representing a ($C_1$-$C_4$)alkylcarbonyl group such as acetyl $CH_3$-C(O)-.

More particularly, the amino polysaccharide(s) of the invention are chosen from the chitosans of formula **($VI_3$)** below, and also the organic or mineral acid salts thereof, and the solvates thereof such as hydrates:

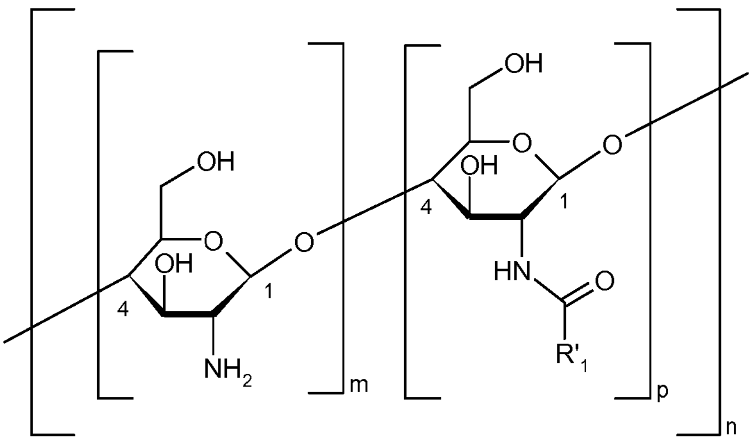

**($VI_3$)**

in which formula **($VI_3$)**:

- **$R'_1$** represents a ($C_1$-$C_4$)alkyl group such as methyl; and
- **n** is an integer greater than or equal to 2, particularly between 3 and 3000 inclusive, more particularly between 5 and 2500, preferentially between 10 and 2300;

- **p** is greater than 0 and ranges up to 0.5, preferably from 0.05 to 0.3, and better still from 0.1 to 0.20 such as 0.15 with m+p being equal to 1;

it being understood that in the chitosan at least one saccharide unit bears an amino group $NH_2$ and at least one other saccharide unit bears a group N(H)-R'$_1$ with R' representing a ($C_1$-$C_4$)alkylcarbonyl group such as acetyl $CH_3$-C(O)-.

For example, when m = 0.7, p = 0.3 this means that 70% of the amine groups are free (unsubstituted) and 30% of the amino groups are N-alkyl($C_1$-$C_4$)carbonyl groups, in particular N-acetyl groups, corresponding to the chitosan of formula:

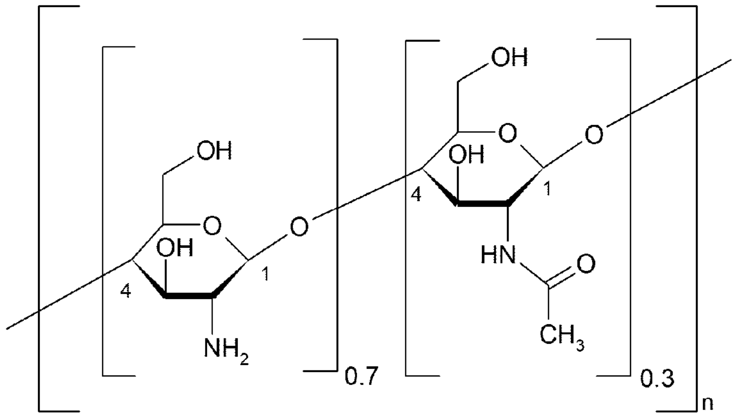

with n as defined above.

According to another form of the invention, the amino polysaccharide(s) of the invention denote a single amino polysaccharide, in particular a chitosan or the organic or mineral acid salts thereof or more particularly the organic acid salts thereof such as the lactic acid salt thereof, the α or β anomers thereof, the optical isomers thereof of L or D configuration, and the solvates thereof such as hydrates.

According to a preferred embodiment, the amino polysaccharide(s) of the invention in particular of formula **(VI)**, **(VI$_1$)**, **(VI$_2$)** or **(VI$_3$)** as defined previously are present in composition (A) or (B) in an amount of between 0.01% and 20% by weight relative to the total weight of the composition which comprises same, more particularly between 0.05% and 10%, more preferentially between 0.1% and 5% by weight relative to the total weight of the composition which comprises same.

According to a particular embodiment, composition (A), (B) or (B') comprises at least one amino acid of formula (III) and/or at least one amino a saccharide of formula (V) or (V') and/or at least one amino polysaccharide, these compounds being present in compositions (A), (B) or (B') in amount(s) of between 0.01% and 20% by weight relative to the total weight of the composition which comprises same, more particularly between 0.05% and 10%, more preferentially between 0.05% and 5% by weight relative to the total weight of the composition which comprises same.

## Alcohols

Compositions (A), (B) or (B') optionally comprise one or more alcohols.

According to another variant, compositions (A), (B) or (B') comprise one or more alcohols.

According to the invention, the term "*alcohol*" means any organic molecule comprising at least one hydroxyl group, other than i) the riboflavins of formula (I), and other than ii) the polymerizable molecules. The alcohols may be aromatic or nonaromatic, saturated or unsaturated, oligomeric or polymeric, and natural or synthetic.

According to a particular embodiment of the invention, composition (A), (B) or (B') of the invention comprises at least one alcohol comprising an optionally substituted (hetero)aryl group, in particular of formula **(IV')**:

$$\text{CYC-(X)}_q\text{-(ALK)}_p\text{-OH} \quad \mathbf{(IV')}$$

and also the organic or mineral acid or base salts thereof, the optical isomers thereof, and the solvates thereof such as hydrates;
in which formula **(IV')**:

- **CYC** represents a heterocycle, cycloalkyl, aryl or heteroaryl group which is optionally substituted, especially with one or more ($C_1$-$C_4$)alkyl or (di)($C_1$-$C_4$)(alkyl)amino groups, preferably aryl such as phenyl or cycloalkyl such as

cyclohexyl,

- **ALK** represents a $(C_1-C_6)$alkylene group,
- **X** represents an oxygen atom, a sulfur atom or a radical $N(R_5)$ with $R_5$ as defined previously, and preferably $R_5$ denoting H or Me: preferably, X represents an oxygen atom;
- **p** and **q**, which may be identical or different, are equal to 0 or 1, it being understood that when p is equal to 0, then q is equal to 0 and that p is equal to 1 when q is equal to 1.

Preferably, the compounds of formula **(IV')** are chosen from benzyl alcohol, phenoxyethanol and *N*,*N*-dimethylaminobenzyl alcohol.
According to another preferred embodiment of the invention, the alcohols are chosen from terpenes, more preferentially chosen from menthol, isoborneol and neomenthol.
According to another particular embodiment, the alcohols are chosen from linear or branched, saturated or unsaturated $(C_1-C_6)$ alkanols, such as isopropanol.
According to another particular embodiment of the invention, composition (A) or (B) of the invention comprises at least one alcohol comprising an optionally substituted (hetero)aryl group, in particular of formula **(IV"):**

$$R_eR_fN\text{-}(ALK')_{p'}\text{- CYC-}(ALK)_p\text{-OH}$$

and also the organic or mineral acid or base salts thereof, the optical isomers thereof, and the solvates thereof such as hydrates;
in which formula **(IV):**

- **CYC** represents an optionally substituted heterocycle, cycloalkyl, aryl or heteroaryl group, preferably aryl such as phenyl,
- **ALK** and **ALK'** independently represents a $(C_1-C_6)$alkylene group,
- $\mathbf{R_e}$ and $\mathbf{R_f}$, which may be identical or different, represent a hydrogen atom or a $(C_1-C_6)$alkyl group such as methyl; and
- **p** and **p'** are independently equal to 0 or 1.

Preferably the compounds of formula **(IV")** are chosen from an N,N-dimethylaminobenzyl alcohol.
According to a preferred embodiment, the alcohol(s), in particular the alcohol(s) of formula (IV') or (IV"), are present in compositions (A), (B) or (B') in an amount of between 0.01% and 20% by weight relative to the total weight of the composition which comprises same, more particularly between 0.05% and 10%, more preferentially between 0.05% and 5% by weight relative to the total weight of the composition which comprises same.
According to a preferred embodiment of the invention, compositions (A), (B) or (B') comprise one or more amines, in particular amino acids, and one or more alcohols as defined previously, in an amount inclusively between 0.01% and 30% by weight relative to the total weight of compositions (A), (B) or (B'), in particular between 0.05% and 20%, more particularly between 0.1% and 10%, preferentially between 1% and 5% by weight relative to the total weight of compositions (A), (B) or (B').

## Step 2) - the step of supplying energy bv light radiation

According to the process of the invention, step 2) comprises the exposure of keratin materials to at least one artificial and/or natural light radiation which may be continuous or non-continuous, before, during or after application of compositions (A), (B) or (B') as defined previously to said materials.
According to the process of the invention, step 2) in particular comprises the exposure of keratin materials to at least one artificial and/or natural light radiation which may be continuous or non-continuous, before, during or after application of compositions (A), (B) or (B') as defined previously to said materials.
For the purposes of the invention, the term "artificial light radiation" means light radiation other than natural daylight (generated by the sun). In other words, natural daylight (generated by the sun) is not artificial light radiation.
The term "*natural radiation*" means radiation whose sole light source is that of the day generated by the sun.
Preferably, the exposure of the keratin fibers to artificial light radiation is performed at a wavelength of between 360 and 600 nm, preferably between 375 and 550 nm, more preferentially between 400 and 480 nm.
Preferably, the artificial light radiation has an amount of energy per unit area of greater than or equal to 1 $J/cm^2$, more preferentially strictly greater than 1 $J/cm^2$, even more preferentially between 1.001 and 100 $J/cm^2$, better still between 2 and 50 $J/cm^2$, particularly preferably between 3 and 10 $J/cm^2$.
Preferably, the artificial light radiation is generated using a device chosen from arc lamps such as xenon lamps and mercury lamps, fluorescent lamps, incandescent lamps such as halogen lamps, light-emitting diodes (LED), organic light-emitting diodes (OLED) and lasers.

Mention may be made, for example, of goLITE BLU from the company Philips, the lamp Energylight HF 3319/01 from the company Philips, the lamps Dayvia White and Messa from the company Solvital, the lamp Lumino Plus from the company Lanaform, the lamp Medibeam from the company Medibeam, the lamp M-LED 01 from the company Meimed, the lamp Lifemax Light Pod from the company Lifemax, the lamp Lite-Pad from the company Reicorp, the lamps Omnilux Clear-U and New-U from the company Omnilux, the 1000 W xenon arc lamp from the company Lot-Oriel and the lamp Camag Box 3 (4x8 W) from the company Camag.

Preferably, the duration of exposure of the keratin materials to artificial or natural light radiation is greater than or equal to 5 seconds, more preferentially between 10 seconds and 60 minutes, even more preferentially between 15 seconds and 50 minutes, and better still between 20 seconds and 40 minutes.

According to a first variant of the invention, the invention relates to a process (1) for treating keratin materials, in particular keratin fibers, especially human keratin fibers such as the hair, comprising:

1) a step of applying a composition **(A)** comprising:

   i) one or more compounds of formula **(I)** as defined previously; and
   ii) one or more polymerizable molecules as defined previously;

2) a step of supplying energy to said keratin materials, which consists in exposing said materials to at least one artificial or natural light radiation as defined previously,

steps 1) and 2) possibly being performed simultaneously or separately, preferably simultaneously or else separately 1) and then 2).

According to another variant of the invention, the invention relates to a process (2) for treating keratin materials, in particular keratin fibers, especially human keratin fibers such as the hair, comprising:

1) a step of applying a composition (B) or (B') as defined previously; and
2) a step of supplying energy to said keratin materials, which consists in exposing said materials to at least one artificial or natural light radiation,

steps 1) and 2) possibly being performed simultaneously with the application, or sequentially, preferably simultaneously with the application or else sequentially 1) and then 2).

Preferably, the process of the invention is process (1).

According to a particular embodiment of the invention, process (1) for treating keratin materials of the invention involves at the same time (simultaneously) steps 1) and 2).

According to one variant, at the same time as the application of composition (A) to the keratin materials, said materials are exposed to at least one artificial and/or natural light radiation.

According to another variant of the invention according to process (2), composition (A) is exposed to at least one artificial and/or natural light radiation to give composition (B), said composition (B) then being applied to the keratin materials.

According to a particular embodiment of the invention, when steps 1) and 2) of the process are performed simultaneously, the duration of exposure of the keratin materials to at least one artificial and/or natural light radiation is between 5 seconds and 3 hours, preferably between 1 minute and 1 hour, such as 15 minutes; optionally followed by a step of rinsing said keratin fibers.

According to a preferred embodiment of the invention, when step 2) is performed after step 1), the duration of exposure of the keratin materials to artificial or natural light radiation is between 5 seconds and 3 hours, preferably between 1 minute and 1 hour, such as 15 minutes; optionally, a step of leaving composition (A) on said keratin fibers for a time of between 5 seconds and 3 hours, preferably between 1 minute and 1 hour, such as 15 minutes, is performed between step 1) and step 2); optionally, a step of rinsing said keratin fibers is performed after step 2).

According to another preferred embodiment of the invention, when step 2) is performed after step 1), the duration of exposure of the keratin materials to artificial or natural light radiation is between 5 seconds and 3 hours, preferably between 1 minute and 1 hour, such as 15 minutes; optionally, a step of leaving composition (A), (B) or (B') on said keratin fibers for a time of between 5 seconds and 3 hours, preferably between 1 minute and 1 hour, such as 15 minutes, is performed between step 1) and step 2); a step of rinsing said keratin fibers is performed after step 1).

Compositions (A), (B) or (B') of the invention may be applied to dry or wet keratin materials, preferably to dry or wet hair, preferably to dry hair.

The bath ratio of the applied compositions may range from 0.1 to 10, more particularly from 0.2 to 5 and preferably between 0.5 and 3. The term "bath ratio" means the ratio between the total weight of the applied composition and the total weight of keratin materials to be treated.

In particular, the step of applying composition (A), (B) or (B') to the keratin materials may be followed by a leave-on time. The leave-on time, i.e. the time of contact of composition (A), (B) or (B') with the keratin materials, is preferably at least 5 minutes, preferably between 10 and 60 minutes and preferably between 15 and 45 minutes.

Rinsing of the hair may optionally be envisaged after applying composition (A), (B) or (B') and optionally the leave-on time.

The hair may then optionally be wrung dry, preferably wrung dry.

**Process for preparing a composition (B')**

According to a preferred embodiment of the invention, the process for preparing composition (B') comprises step a) of mixing i) one or more flavin derivatives chosen from the compounds of formula **(I)** as defined previously with ii) one or more polymerizable molecules chosen from those of formula **(II), (IIa)**, **(IIb)** or **(IIc)** as defined previously and with iii) one or more amines and/or one or more alcohols other than the riboflavins of formula **(I)** and other than the polymerizable molecules of formula **(II), (IIa)**, **(IIb)** or **(IIc)**, preferably one or more amines chosen from amino acids; followed by a step b) of exposing said mixture to at least one artificial and/or natural light radiation, in particular for at least 10 minutes.

More particularly, the process for preparing composition (B') comprises step a) of mixing i) one or more flavin derivatives chosen from the compounds of formula (I) as defined previously with ii) one or more polymerizable molecules chosen from those of formula **(II)**, **(IIa)**, **(IIb)** or **(IIc)** as defined previously and with iii) one or more amino acids preferably of formula **(III)** as defined previously; followed by a step b) of exposing said mixture to at least one artificial and/or natural light radiation, in particular radiation, for at least 2 minutes, preferably for between 2 minutes and up to 10 hours, more preferentially between 15 minutes and 5 hours, such as 3 hours.

A subject of the invention is also composition (B') obtained via the process as described previously.

The process of the invention is preferably performed on keratin fibers, especially human keratin fibers, such as the hair.

According to one variant, the process of the invention is performed on keratin fibers, especially human keratin fibers, such as the hair. According to a particular variant of the invention, the process is performed on damaged, bleached or sensitized keratin fibers.

According to a particular form of the invention, the process of the invention is a process for treating keratin materials, in particular keratin fibers, especially human keratin fibers such as the hair, comprising:

1) a step of applying to dry or wet keratin materials one or more compounds of formula **(I)** as described previously, or a composition (A), (B) or (B') containing the compounds of formula **(I)**, optionally in the presence of one or more amines and/or of one or more alcohols as defined previously; and then

  1i) optionally a step of leaving on the keratin materials, preferably for at least 1 minute, preferably for a time of between 5 and 60 minutes, more preferentially between 10 and 45 minutes; and then
  1ii) optionally wringing out, and then
  1iii) optionally drying in the open air or with a heating device which delivers heat of less than or equal to 80°C, for example using a hood or a hairdryer, and then

2) a step of supplying energy to said keratin materials, which consists in exposing said materials to at least one artificial or natural light radiation; and then
2i) a rinsing step.

According to another variant, the process is performed on "natural" keratin fibers, i.e. fibers that have not undergone any chemical treatment such as dyeing, bleaching or permanent-waving.

**Compositions (A), (B)** and **(B')**

*The compositions*

The composition(s) of the invention are cosmetic, i.e. they contain a physiologically acceptable medium, that is to say a medium that is compatible with human keratin materials such as the skin (of the body, face, around the eyes or the scalp), the hair, the eyelashes, the eyebrows, bodily hair, the nails or the lips.

The physiologically acceptable medium of the composition(s) used in the process according to the invention is advantageously an aqueous medium. It may be constituted, for example, of water or of a mixture of water and of at least one cosmetically acceptable organic solvent. Examples of organic solvents that may be mentioned include $C_2$-$C_4$

lower alcohols, such as ethanol and isopropanol; polyols, especially those containing from 2 to 6 carbon atoms, for instance glycerol, propylene glycol, butylene glycol, pentylene glycol, hexylene glycol, dipropylene glycol or diethylene glycol; polyol ethers, for instance 2-butoxyethanol, propylene glycol monomethyl ether and diethylene glycol monomethyl ether or monoethyl ether; and mixtures thereof.

The cosmetic compositions of the invention are preferably water-based and then comprise water at a concentration ranging from 10% to 99.5%, better still from 30% to 95% and even better still from 50% to 95% by weight relative to the total weight of the composition, and even more preferentially from 60% to 90% by weight relative to the total weight of the composition.

The composition used according to the invention may also contain one or more cosmetic additives chosen from nonionic, anionic, cationic and amphoteric surfactants, vitamins and provitamins other than the compounds of formula (I) including panthenol, fillers, dyestuffs, nacreous agents, opacifiers, sequestrants, film-forming polymers, cationic, anionic or neutral polymers, associative polymers, plasticizers, silicones, thickeners, oils, antifoams, moisturizers, emollients, penetrants, fragrances and preserving agents; preferably one or more nonionic, anionic, cationic or amphoteric surfactants, cationic, anionic or neutral polymers, or associative polymers.

Depending on their nature and the purpose of the composition, the usual cosmetic ingredients may be present in usual amounts, which can be readily determined by those skilled in the art and which may be, for each ingredient, between 0.01% and 80% by weight. Those skilled in the art will take care to select the ingredients included in the composition, and also the amounts thereof, so that they do not harm the properties of the compositions of the present invention.

These compositions may be packaged in pump-action bottles or in aerosol containers, so as to apply the composition in vaporized (lacquer) form or in the form of a mousse. Such packaging forms are indicated, for example, when it is desired to obtain a spray or a mousse, for treating the hair. In these cases, the composition preferably comprises at least one propellant.

*pH of the compositions:*

The pH of compositions (A), (B) or (B') is preferably between 3 and 10, more particularly in the region of neutrality between 6 and 8, such as 7.

The pH values may be adjusted with an organic or mineral acid, or with an alkaline agent chosen from mineral or organic or hybrid alkaline agents or mixtures thereof.

The term "*organic acid*" means an acid, i.e. a compound that is capable of releasing a cation or proton $H^+$ or $H_3O^+$ in aqueous medium, which comprises at least one optionally unsaturated, linear or branched $C_1$-$C_{20}$ hydrocarbon-based chain, a (hetero)cycloalkyl or (hetero)aryl group and at least one acidic chemical function chosen in particular from carboxyl C(O)OH, sulfonic $SO_3H$, sulfinic $SO_2H$, phosphonic $PO_3H_2$ and phosphinic $PO_2H_2$.

More particularly, the acids used are chosen from hydrochloric acid HCl, hydrobromic acid HBr, sulfuric acid $H_2SO_4$, alkylsulfonic acids: $(C_1$-$C_6)$Alk-$S(O)_2OH$ such as methylsulfonic acid and ethylsulfonic acid; arylsulfonic acids: Ar-$S(O)_2OH$ such as benzenesulfonic acid and toluenesulfonic acid; $(C_1$-$C_6)$alkoxysulfinic acids: Alk-O-S(O)OH such as methoxysulfinic acid and ethoxysulfinic acid; aryloxysulfinic acids such as tolueneoxysulfinic acid and phenoxysulfinic acid; phosphoric acid $H_3PO_4$; triflic acid $CF_3SO_3H$ and tetrafluoroboric acid $HBF_4$, and carboxylic acid(s) of formula **(D)** below:

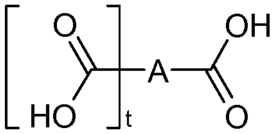

**(D)**

in which formula **(D)** or a salt thereof:

**A** represents a saturated or unsaturated, cyclic or non-cyclic, and aromatic or non-aromatic hydrocarbon-based group, which is monovalent when t is 0 or polyvalent when t is greater than or equal to **1**, comprising from 1 to 50 carbon atoms, which is optionally interrupted with one or more heteroatoms and/or optionally substituted, especially with one or more hydroxyl groups; preferably, A represents a monovalent $(C_1$-$C_6)$alkyl group or a polyvalent $(C_1$-$C_6)$alkylene group optionally substituted with one or more hydroxyl groups.

Particularly, the carboxylic acid(s) of formula **(D)** as defined previously, and preferably the acid(s) used, are an alpha-hydroxy acid such as lactic acids, glycolic acids, tartaric acids or citric acids.

The mineral alkaline agent(s) are preferably chosen from aqueous ammonia, alkaline carbonates or bicarbonates such as sodium or potassium carbonates and sodium or potassium bicarbonates, sodium hydroxide or potassium

hydroxide, or mixtures thereof.

According to an advantageous embodiment of the invention, the alkaline agent(s) are organic amines, i.e. they contain at least one substituted or unsubstituted amino group.

The organic alkaline agent(s) are more preferentially chosen from organic amines with a $pK_b$ at 25°C of less than 12, preferably of less than 10 and even more advantageously of less than 6. It should be noted that it is the $pK_b$ corresponding to the function of highest basicity.

Hybrid compounds that may be mentioned include the salts of the amines mentioned previously with acids such as carbonic acid or hydrochloric acid.

The organic alkaline agent(s) are chosen, for example, from alkanolamines, oxyethylenated and/or oxypropylenated ethylenediamines, amino acids and the compounds of formula **(E)** below:

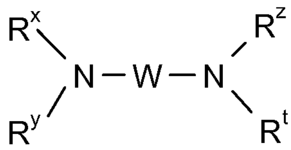

**(E)**

in which formula **(E):**

- **W** is a divalent $C_1$-$C_6$ alkylene radical optionally substituted with a hydroxyl group or a $C_1$-$C_6$ alkyl radical, and/or optionally interrupted with one or more heteroatoms such as oxygen or $NR^u$;
- $\mathbf{R^x}$, $\mathbf{R^y}$, $\mathbf{R^z}$, $\mathbf{R^t}$ and $\mathbf{R^u}$, which may be identical or different, represent a hydrogen atom or a $C_1$-$C_6$ alkyl, $C_1$-$C_6$ hydroxyalkyl or $C_1$-$C_6$ aminoalkyl radical.

Preferably, the alkanolamine is ethanolamine (or monoethanolamine).

In one variant of the invention, the composition comprises, as alkaline agent, one or more alkanolamines (preferably ethanolamine) and aqueous ammonia. In this variant, the alkanolamine(s) are present in a predominant amount relative to the aqueous ammonia.

The examples that follow serve to illustrate the invention without, however, being limiting in nature.

## EXAMPLES

The process according to the invention makes it possible to obtain polymeric compounds which may be generated in keratin fibers or else externally to keratin fibers and then applied to said fibers.

*Example 1*

*Preparation of a composition (B) or (B') intended to be applied to keratin fibers:*

An aqueous composition constituted of sodium riboflavin 5'-monophosphate hydrate (3 g%), L-arginine (3 g%), itaconic acid (38 g%) and water (pH adjusted to 7 with NaOH) was irradiated with a 1000 W xenon arc lamp for 3 hours to create a polymer composition (B), which was then applied to the fiber.

*Example 2*

The following compositions were prepared according to the following table:

| Locks* | g% ingredients | | | | Irradiation |
|---|---|---|---|---|---|
| | Sodium salt of riboflavin 5'-monophosphate hydrate | L-arginine | Itaconic acid | | |
| **1 (comparative)** | - | - | - | - | NO |
| **2 (comparative)** | - | - | - | - | YES |
| **3 (comparative)** | 3 | - | - | - | NO |
| **4 (comparative)** | - | 3 | - | - | NO |

(continued)

| Locks* | g% ingredients | | | | Irradiation |
|---|---|---|---|---|---|
| | Sodium salt of riboflavin 5'-monophosphate hydrate | L-arginine | Itaconic acid | | |
| **5 (comparative)** | - | - | - | 40 | NO |
| **6 (comparative)** | 3 | - | - | - | YES |
| **7 (comparative)** | - | 3 | - | - | YES |
| **8 (comparative)** | - | - | - | 40 | YES |
| **9 (comparative)** | 3 | 3 | - | - | NO |
| **10 (comparative)** | 3 | - | - | 40 | NO |
| **11 (comparative)** | - | 3 | - | 40 | NO |
| 12 **(comparative)** | 3 | 3 | - | - | YES |
| **13 (comparatif)** | - | 3 | - | 40 | YES |
| **14 (invention)** | 3 | - | - | 40 | YES |
| **15 (comparative)** | 3 | 3 | - | 40 | NO |
| **16 (invention)** | 3 | 3 | - | 40 | YES |
| **17 (comparative)** | - | - | 1 | - | NO |
| **18 (comparative)** | - | - | 1 | - | YES |
| **19 (invention)** | 0.1 | 0.1 | 1 | - | YES |

* The locks numbered 1 to 19 are made using locks of damaged hair (bleached hair). The word "NO" implies that the processes were performed without natural daylight and without artificial light.

*Preparation of the samples*

2.7 g of locks of 20 cm SA20 bleached hair, which were washed and dried beforehand, were used. 1.5 g of each formula were applied for each gram of dry hair. The samples were left on for 15 minutes, wrapped in a thin transparent plastic film. The film was removed and the hair was irradiated using a 1000 W xenon arc lamp for 15 minutes, and then rinsed with excess water at 37°C, combed and dried under a hood at 60°C for 10 minutes. The placebo samples were prepared in an identical manner, except that the sample was covered with aluminum foil instead of transparent film (to protect it from irradiation). After drying, the samples were combed (comb passed through five times) and then left suspended vertically.

*Test method*

*Effect of a ponytail*

The more the hair is damaged, the more the folding of the sample remains marked on the hair after the stress has been removed.

The locks were folded in two (the root toward the end of the hair) and fixed using an elastic band so as to make a loop of about 3 cm, i.e. the length of the loop is 3 cm or, in other words, the elastic band is 3 cm from the bottom of the loop. The looped locks were suspended with the loop at the bottom in a glove box at 80% RH and 26°C for 2 hours. The locks were removed from the glove box, the elastic band was removed and the locks were suspended vertically. The fold angle was measured according to the reporter's principle (see Fig. 1) immediately after removing the elastic band (initial angle) and then at the end of the study (final angle after 14 hours).

*Results*

Angle reduction = final angle - initial angle

The greater the angle reduction, the more the fold imprint disappears and the more longitudinal the lock.

| | Initial angle (°) | Final angle (°) | Angle reduction |
|---|---|---|---|
| **1 (comparative)** | 130 | 140 | 10 |
| **2 (comparative)** | 140 | 150 | 10 |
| **3 (comparative)** | 130 | 130 | 0 |
| **4 (comparative)** | 130 | 140 | 10 |
| **5 (comparative)** | 130 | 140 | 10 |
| **6 (comparative)** | 130 | 140 | 10 |
| **7 (comparative)** | 130 | 130 | 0 |
| **8 (comparative)** | 120 | 130 | 10 |
| **9 (comparative)** | 130 | 140 | 10 |
| **10 (comparative)** | 130 | 140 | 10 |
| **11 (comparative)** | 130 | 150 | 20 |
| **12 (comparative)** | 120 | 130 | 10 |
| **13 (comparative)** | 130 | 140 | 10 |
| **14 (invention)** | 140 | 170 | 30 |
| **15 (comparative)** | 130 | 140 | 10 |
| **16 (invention)** | 140 | 180* | 40* |
| **17 (comparative)** | 130 | 140 | 10 |
| **18 (comparative)** | 130 | 140 | 10 |
| **19 (invention)** | 140 | 170 | 30 |
| * Maximum relaxation (no fold present) | | | |

After removing the elastic band, the locks numbered 14, 16 and 19 (present invention) show a better return longitudinal shape, i.e. the fold imprint is no longer visible on these locks. This is not the case for the other comparative locks (1 to 13 and 15, 17 and 18). In addition, it is seen that the damaged locks treated via the process of the invention are perceived as being identical to a natural lock, indicating that the process of the invention has repaired the damaged hair.

*Example 3*

Comparative study (vs. WO2016/126121)

The use of the same yeast (Saccharomyces cerevisiae) as described in WO2016/126121 is not possible since the details of the supplier are not given neither any procedure to prepare the yeast nor the extraction procedure of the riboflavin. After a literature search it was concluded that the concentration of riboflavin in S. cerevisiae is between 0.8-1.5 $\mu$M. Thus the higher concentration of 1.5 $\mu$M has been used to do the comparative study. Therefore, a composition comprising 20 % by weight of S. cerevisiae comprises an equivalent quantity of riboflavin of 1.13g $\times$ $10^{-4}$ % by weight. A particular reference for the cytosolic concentration of riboflavin in S. cerevisiae is Biosynthesis of Vitamin B2 (Riboflavin), Annual Review of Nutrition, Vol. 20:153-167 (Volume publication date July 2000) - https://doi.org/10.1146/annurev.nutr.20.1.153

In this study, pentaerythritol tetraacrylate has been used as a polymerizable molecule:

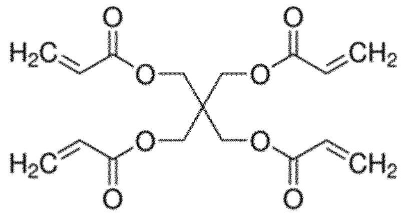

The following formulations were prepared. The ingredients were mixed, stirred for one hour in an closed amber vial before use.

| Formulation Number | Riboflavin[1] | Pentaerythritol tetraacrylate[2] | Urea[3] | Benzophenone 4 | EtOH | Qsp 5% Triton X-100 [5] in Water |
|---|---|---|---|---|---|---|
| 1 | 19 | 5g | - | - | 50g | 100g |
| 2 | 1g | - | 0.5g | 0.5g | 50g | 100g |
| 3 | $1.13 \times 10^{-4}$ g | - | 0.5g | 0.5g | 50g | 100g |
| [1]Sigma-Aldrich - Ref. R7649<br>[2]Sigma-Aldrich - Ref. 408263<br>[3]Sigma-Aldrich - Ref. U4884<br>[4]BASF- Ref. Uvinul 40<br>[5]Sigma-Aldrich - Ref. X-100 | | | | | | |

Preparation of hair swatches

| Example | Formulation number | Irradiation |
|---|---|---|
| 1 (Invention) | 1 | Natural light |
| 2 (Invention) | 1 | Artificial light |
| 3 (Comparative) | 2 | Natural light |
| 4 (Comparative) | 2 | Artificial light |
| 5 (Comparative) | 3 | Natural light |
| 6 (Comparative) | 3 | Artificial light |

The hair swatches (2.7g, 27cm length) were placed on a piece of aluminium foil. 5.4g of each formulation was applied onto each hair swatch. The swatches were covered with a transparent plastic film to reduce evaporation. Examples 1, 3, and 5 were exposed to natural light for 60 minutes. After 30 of the 60 minutes the swatches were turned over to ensure each side of the swatches was exposed to at least 30 minutes of natural light. The irradiance of natural light was measured at 64microW/$cm^2$ using a Thor PM100D Optical Power Meter.

Examples 2, 4 and 6 were irradiated with a home-made array of LED lights emitting light at 450 nm for 60 minutes. The array of LEDs were positioned 5 cm above the swatch and the irradiance measured at 26 mW/$cm^2$). After 30 of the 60 minutes the swatches were turned over to ensure each side of the swatches was irradiated to at least 30 minutes.

After exposure to the light all the swatches were suspended vertically, covered with a towel and left to dry.

Shampooing

The swatches were subjected to 5 repeat washes. Washing consisted of placing the swatch in a bath containing 100mL of 3% sodium lauryl ether sulfate (SLES) for 1 minute then rinsing for 10 seconds under tap water. The process is repeated 4 times using fresh SLES solution each time. At the end of the fifth wash and rinse the swatches were dried in a professional helmet hairdryer at 60°C.

Hair repair test - repeat combing (anti breakage)

The swatches were suspended vertically and combed 30 times with a fine toothed comb at a speed of 30cm/sec. The amount of broken hair was collected and weighed. The lower the weight of hair collected the better the formulation is at reducing the breakage of hair i.e. repairing the hair.

Results

| Example | Amount of broken hair (g) |
|---|---|
| 1 (Invention) | 0,0813 |
| 2 (Invention) | 0,0532 |
| 3 (Comparative) | 0,1336 |
| 4 (Comparative) | 0,1174 |
| 5 (Comparative) | 0,1683 |
| 6 (Comparative) | 0.1749 |

The results show that hair treated according to Examples 1 and 2 with 1% riboflavin and pentaerythritol tetraacrylate have been repaired since there is less hair breakage during the repeated combing. Example 2 using the LED array gave better results than natural light.

**Claims**

**1.** A process for treating keratin materials, in particular keratin fibers, especially human keratin fibers such as the hair, comprising:

1) a step of applying a composition (A) comprising:

i) one or more flavin derivatives chosen from the compounds of formula (I) below:

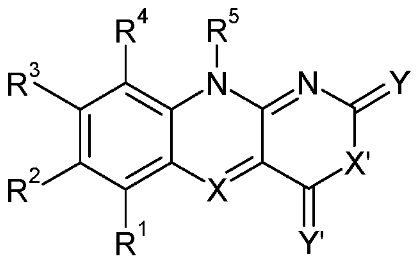

**(I)**

and also the organic or mineral acid or base salts thereof, the optical isomers, geometrical isomers and tautomers thereof, and the solvates thereof such as hydrates; in which formula **(I)**:

• $\mathbf{R^1}$, $\mathbf{R^2}$, $\mathbf{R^3}$ and $\mathbf{R^4}$, which may be identical or different, represent a hydrogen atom, a halogen atom or a group chosen from hydroxyl, $(C_1\text{-}C_6)$alkyl, $(C_1\text{-}C_6)$alkoxy, $(C_1\text{-}C_6)$alkylthio, (di)$(C_1\text{-}C_6)$(alkyl)amino, nitro(so), in particular chosen from hydrogen and $(C_1\text{-}C_6)$alkyl; more particularly, $R^1$ and $R^4$ represent a hydrogen atom and $R^2$ and $R^3$ represent a hydrogen atom or a $(C_1\text{-}C_4)$alkyl group such as methyl;
• $\mathbf{R^5}$ represents a hydrogen atom or a $(C_1\text{-}C_8)$alkyl group optionally substituted with one or more groups chosen from i) hydroxyl, ii) $R^6$-C(Y")-O- with $R^6$ representing a hydrogen atom or a $(C_1\text{-}C_4)$alkyl or aryl$(C_1\text{-}C_4)$alkyl group such as benzyl and Y" represents an oxygen or sulfur atom, or N($R^7$) with $R^7$

representing a hydrogen atom or a $(C_1-C_4)$alkyl group, Y" preferably representing an oxygen atom, iii) phosphoric $(HO)_2P(O)-O-$, iv) $-O-[(HO)P(O)-O]_n-$Suc-Het with Suc representing a divalent sugar group such as ribose, and Het representing a heteroaryl group such as adenine, n is an integer equal to 0, 1 or 2, preferably 2; in particular, the group Suc-Het represents a ribose group substituted with an adenine group;

• **X** represents a nitrogen atom or a methylene group $C(R^8)$ with $R^8$ representing a hydrogen atom or a $(C_1-C_4)$alkyl group; preferably, X represents a nitrogen atom;

• **X'** represents an oxygen atom or a group $NR^9$ with $R^9$ representing a hydrogen atom or a $(C_1-C_6)$alkyl group; in particular, X' denotes $NR^9$, preferably NH;

• **Y** and **Y'**, which may be identical or different, represent an oxygen or sulfur atom or a group $NR^{10}$ with $R^{10}$ representing a hydrogen atom or a $(C_1-C_6)$alkyl group, preferably Y and Y' represent an oxygen atom; and

ii) one or more polymerizable molecules; and

2) a step of supplying energy to said keratin materials, which consists in exposing said materials to at least one artificial or natural light radiation,
it being understood that:

- composition (A) may undergo a step of energy supply before being applied to the keratin materials, which step consists in exposing said composition to at least one artificial or natural light radiation to lead to a composition (B) which will then be applied to said materials;
- steps 1) and 2) are possibly performed simultaneously with the application, or sequentially, preferably simultaneously with the application or else sequentially to the application 1) and then 2);
- the polymerizable molecule(s) include at least one insaturated group wherein:

■ the unsaturated group is $-C{\equiv}CR'$ or $-CR{=}CR'_2$ with R and R', which may be identical or different, representing a hydrogen atom, a $(C_1-C_4)$alkyl group such as methyl or carboxyl (COOH), preferably a hydrogen atom or a $(C_1-C_4)$alkyl group such as methyl; **or**

■ the polymerizable molecule(s) are chosen from those of formula (II) below:

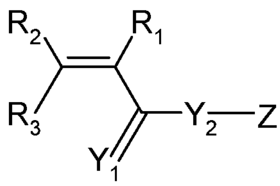

**(II)**

and also the organic or mineral acid or base salts thereof, the optical isomers, geometrical isomers and tautomers thereof, and the solvates thereof such as hydrates; in which formula **(II)**:

• $\mathbf{R_1}$ and $\mathbf{R_2}$, which may be identical or different, represent a hydrogen atom or a $(C_1-C_8)$alkyl or $(C_2-C_8)$alkenyl group, said alkyl or alkenyl groups being:

- optionally interrupted with one or more heteroatoms such as O, S and $N(R_5)$, with $R_5$ representing a hydrogen atom or a $(C_1-C_4)$alkyl group such as methyl, and/or
- optionally substituted with one or more radicals chosen from the following radicals: hydroxyl, $(di)(C_1-C_4)(alkyl)amino$, $(C_1-C_4)$alkoxy and $-C(Y'_1)-Y'_2-R'_4$ such as carboxyl;

• $\mathbf{R_3}$ is as defined for $R_2$, or else represents a group $-C(Y'_1)-Y'_2-R'_4$;

• $\mathbf{Y_1}$, $\mathbf{Y_2}$, $\mathbf{Y'_1}$ and $\mathbf{Y'_2}$, which may be identical or different, represent an oxygen or sulfur atom or $N(R_5)$, with $R_5$ representing a hydrogen atom or a $(C_1-C_4)$alkyl group, preferably, $Y_1$ and $Y'_1$ represent an oxygen atom and $Y_2$ and $Y'_2$ represent an oxygen atom or a group $N(R_5)$; more particularly, $Y_2$ and $Y'_2$ represent an oxygen atom; and

• $\mathbf{R_4}$ and $\mathbf{R'_4}$, which may be identical or different, represent a hydrogen atom or a $(C_1-C_{16})$alkyl group optionally substituted with one or more radicals, which may be identical or different, chosen

from the following radicals: a) amino ($NH_2$), mono- or di($C_1$-$C_4$)alkylamino such as dimethylamino, b) hydroxyl (OH), c) $SO_3H$, d) carboxyl ($CO_2H$), e) cyano, f) (poly)(halo)($C_1$-$C_4$)alkyl such as trifluoromethyl ($CF_3$), g) ($C_1$-$C_4$)alkoxycarbonyl; said alkyl radical being optionally interrupted with one or more heteroatoms such as O, S and N($R_5$) with $R_5$ as defined previously, and preferably $R_5$ denoting H or Me;

• Z denotes a radical $R_4$ as defined previously or a radical **(Z')** of formula:

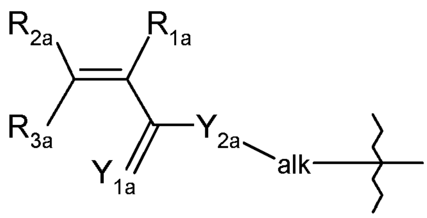

(**Z'**)

in which formula **(Z')**:

- $Y_{1a}$, $Y_{2a}$, $R_{1a}$, $R_{2a}$ and $R_{3a}$ have, respectively and independently, the same meaning as $Y_1$, $Y_2$, $R_1$, $R_2$ and $R_3$ defined previously;
- alk denotes a divalent ($C_1$-$C_{16}$)alkylene radical optionally interrupted with one or more oxygen or sulfur atoms or with one or more NH groups, said alkylene radical being optionally substituted with one or more radicals chosen from hydroxyl and a radical of formula **(Z"):**

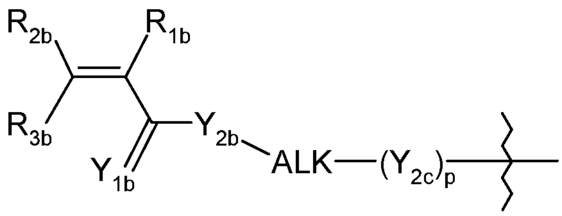

(**Z"**)

in which formula **(Z')**:

- $Y_{1b}$, $Y_{2b}$, $Y_{2c}$, $R_{1b}$, $R_{2b}$ and $R_{3b}$ have, respectively and independently, the same meaning as $Y_1$, $Y_2$, $R_1$, $R_2$ and $R_3$ defined previously, ALK denoting a ($C_1$-$C_6$)alkylene radical (divalent radical) such as methylene or propylene and p being equal to 0 or 1;
-

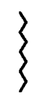

denoting the point of attachment between alk and $Y_2$ in the case of formula (Z') and
-

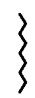

denoting the point of attachment between $(Y_2)p$ and alk in the case of formula (Z").

2. The process as claimed in the preceding claim, in which the compound(s) of formula **(I)** are such that $\mathbf{R^1}$, $\mathbf{R^2}$, $\mathbf{R^3}$ and $\mathbf{R^4}$, which may be identical or different, represent a hydrogen atom, $(C_1-C_6)$alkyl, $(C_1-C_6)$alkoxy or (di)$(C_1-C_4)$(alkyl)amino, in particular chosen from hydrogen and $(C_1-C_4)$alkyl; preferably, $R^1$ and $R^4$ represent a hydrogen atom and $R^2$ and $R^3$ represent a hydrogen atom or a $(C_1-C_4)$alkyl group such as methyl, preferably a $(C_1-C_4)$alkyl group such as methyl.

3. The process as claimed in either of the preceding claims, in which the compound(s) of formula **(I)** are such that $R^5$ represents a hydrogen atom or a $(C_1-C_6)$alkyl group such as methyl.

4. The process as claimed in either of claims 1 and 2, in which the compound(s) of formula **(I)** are such that $\mathbf{R^5}$ represents a $(C_1-C_6)$alkyl group substituted with one or more groups, which may be identical or different, chosen from i) hydroxyl, ii) $R^6$-C(O)-O-with $R^6$ representing a hydrogen atom, a $(C_1-C_4)$alkyl group such as methyl or n-propyl; iii) phosphoric $(HO)_2P(O)$-O-, iv) -O-[(HO)P(O)-O]$_n$-Suc-Het with Suc representing a monosaccharide such as ribose, and Het representing an optionally substituted heteroaryl group, in particular optionally substituted pyrimidyl-dione such as adenine, n is an integer equal to 1 or 2, preferably 2; in particular, Suc-Het represents a ribose group, preferably α-D-ribose, optionally substituted with an adenine group.

5. The process as claimed in any one of the preceding claims, in which the compound(s) of formula **(I)** are such that, taken together or separately, **X** represents a nitrogen atom; **X**' represents a group $NR^9$ with $R^9$ representing a hydrogen atom or a $(C_1-C_6)$alkyl group; in particular, $NR^9$ preferably NH; and **Y** and **Y**' are identical and more particularly represent an oxygen or sulfur atom; preferably, Y and Y' represent an oxygen atom.

6. The process as claimed in any one of the preceding claims, in which the compound(s) of formula **(I)** are chosen from compounds **1** to **7** below:

| Structure | |
|---|---|
| 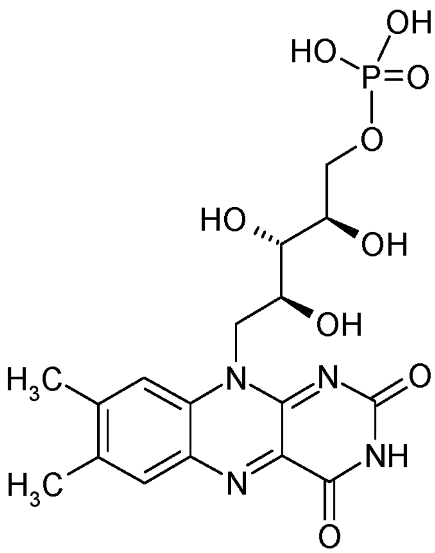 (2R,3S,4S)-5-(7,8-dimethyl-2,4-dioxo-3,4-dihydrobenzo[g]pteridin-10(2H)-yl)-2,3,4-trihydroxypentyl dihydrogen phosphate or riboflavin-5'-phosphate | 1 |

(continued)

| Structure | |
|---|---|
| 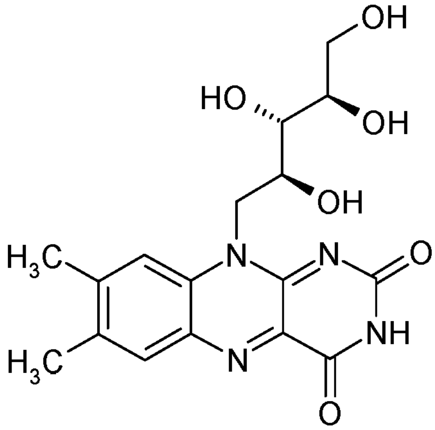<br>7,8-dimethyl-10-((2*R*,3*R*,4*S*)-2,3,4,5-tetrahydroxypentyl)benzo[g]pteridine-2,4-(3H,10H)-dione, or vitamin $B_2$, lactoflavin | 2 |
| | 3 |
| 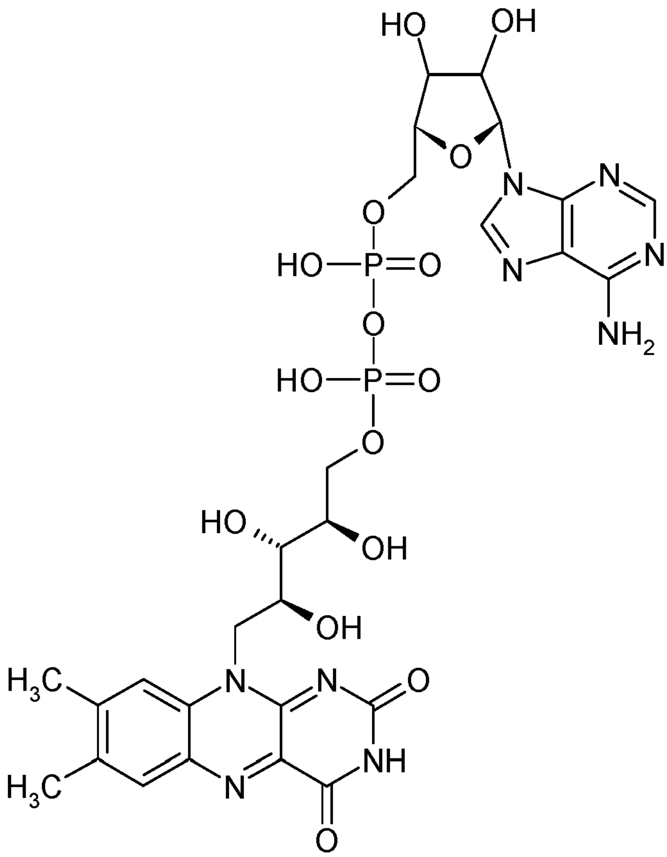<br>Flavin adenine dinucleotide (FAD) | |
| 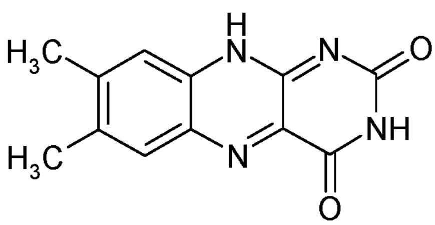 | 4 |

(continued)

| Structure | |
|---|---|
| 7,8-dimethylbenzo[g]pteridine-2,4(3H, 10H)-dione | |
| 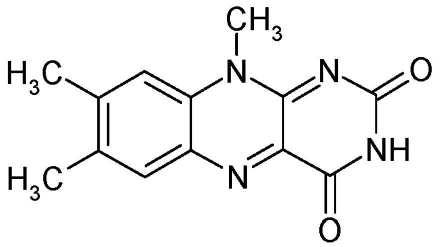 7,8,10-trimethylbenzo[g]pteridine-2,4(3H,10H)-dione | 5 |
| 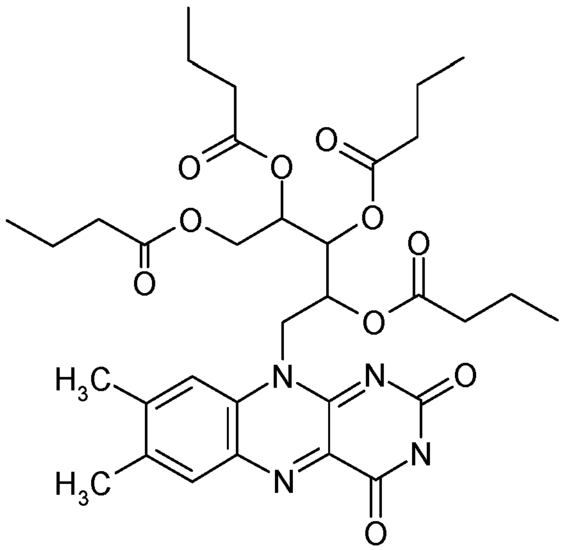 Riboflavin tetrabutyrate | 6 |
| 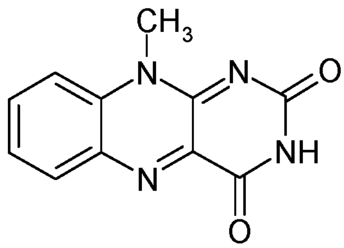 10-methylbenzo[g]pteridine-2,4(3H, 10H)-dione | 7 |

and also the organic or mineral acid or base salts thereof, the optical isomers and tautomers thereof, and the solvates thereof such as hydrates, in particular the salt thereof with an alkali metal such as sodium.

7. The process as claimed in any one of the preceding claims, in which the compound(s) of formula **(I)** are in an amount inclusively between 0.01% and 30% by weight relative to the total weight of composition (A), (B) or (B'), in particular between 0.05% and 20%, more particularly between 0.1% and 10%, preferentially between 1% and 5% by weight relative to the total weight of composition (A) or (B).

8. The process as claimed in any one of the preceding claims, in which ii) the polymerizable molecule(s) have a molecular weight of less than or equal to 500 g/mol, preferably a molecular weight inclusively between 26 and 400 g/mol.

9. The process as claimed in any one of the preceding claims, in which $\mathbf{R_1}$ represents a hydrogen atom, a $(C_1\text{-}C_4)$alkyl

group such as methyl or a ($C_1$-$C_4$)alkyl group substituted with a radical chosen from hydroxyl and -C(Y'$_1$)-Y'$_2$-R'$_4$, in particular -C(O)-OR'$_4$ with R'$_4$ as defined in the preceding claim; preferably, $\mathbf{R_1}$ is a ($C_1$-$C_4$)alkyl group substituted with a carboxyl group such as carboxymethyl.

**10.** The process as claimed in any one of the preceding claims, in which the polymerizable molecule(s) are chosen from those of formula **(IIa)** below:

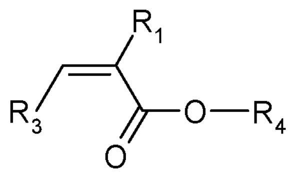

and also the organic or mineral acid or base salts thereof, the optical isomers, geometrical isomers and tautomers thereof, and the solvates thereof such as hydrates;
in which formula **(IIa)** $\mathbf{R_1}$, $\mathbf{R_3}$ and $\mathbf{R_4}$ are as defined in claims 5 to 9;
preferably, the polymerizable molecule(s) ii) are chosen from (($C_1$-$C_4$)alkyl)acrylic acid such as methacrylic acid, acrylic acid or itaconic acid.

**11.** The process as claimed in any one of the preceding claims, in which the polymerizable molecule(s) are chosen from those of formula **(IIb)** below:

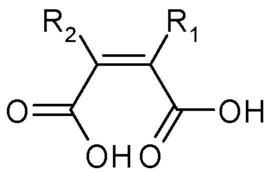

**(IIb)**

and also the organic or mineral acid or base salts thereof, the optical isomers, geometrical isomers and tautomers thereof, and the solvates thereof such as hydrates;
in which formula **(IIb)** $\mathbf{R_1}$ and $\mathbf{R_2}$ are as defined in claims 5 to 10, in particular chosen from hydrogen and ($C_1$-$C_4$)alkyl such as methyl, preferably hydrogen.

**12.** The process as claimed in any one of the preceding claims, in which the polymerizable molecule(s) are chosen from those of formula **(IIc)** below:

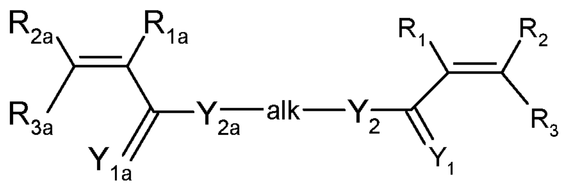

**(IIc)**

and also the organic or mineral acid or base salts thereof, the optical isomers, geometrical isomers and tautomers thereof, and the solvates thereof such as hydrates;
in which formula **(IIc)** $\mathbf{R_1}$, $\mathbf{R_2}$, $R_3$, $Y_1$, $Y_2$, alk, $Y_{2a}$, $Y_{1a}$, $R_{1a}$, $R_{2a}$ and $R_{3a}$ are as defined in claims 5 to 11, in particular such that $R_1 = R_{1a}$ and $R_2 = R_{2a}$ and $R_3 = R_{3a}$ and $Y_1= Y_{1a}$ and $Y_2=Y_{2a}$;
preferentially chosen from compounds (a) to (d) below:

| | |
|---|---|
| 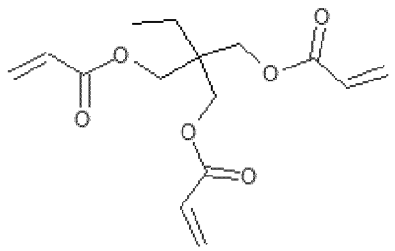 | 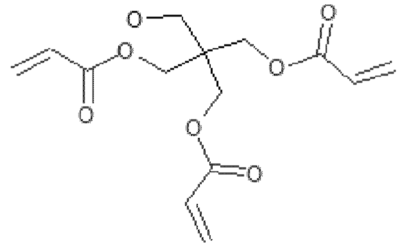 |
| **(a)** | **(b)** |
| 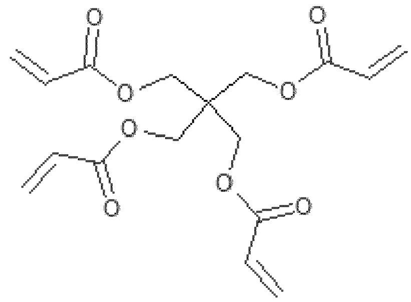 | 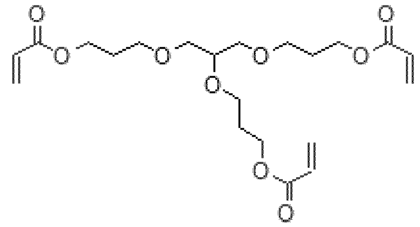 |
| **(c)** | **(d)** |

**13.** The process as claimed in any one of the preceding claims, in which the polymerizable molecule(s) are in an amount inclusively between 0.01% and 50% by weight relative to the total weight of compositions (A) or (B), in particular between 0.1% and 40%, more particularly between 1% and 30%, preferentially between 2% and 20%, more preferentially between 5% and 10% by weight relative to the total weight of compositions (A) or (B).

**14.** The process as claimed in any one of the preceding claims, in which composition (A) or (B) comprises one or more amines other than i) the riboflavins of formula **(I**), and other than ii) the polymerizable molecules; said amines possibly being aromatic or non-aromatic, saturated or unsaturated, oligomeric or polymeric, and natural or synthetic; preferably, the amine(s) are amino acids, chosen from the compounds of formula **(III)** below:

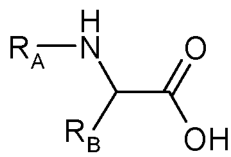

**(III)**

and also the organic or mineral acid or base salts thereof, the optical isomers, geometrical isomers and tautomers thereof, and the solvates thereof such as hydrates;

- **$R_A$** represents a hydrogen atom, a $(C_1$-$C_4)$alkyl or $(C_1$-$C_4)$alkylcarbonyl group or else forms, together with the radical $R_B$ and the nitrogen and carbon atom that bear them, respectively, a 5- to 10-membered monocyclic or bicyclic heterocycle such as pyrrolidinyl; preferably, $R_A$ represents a hydrogen atom;
- **$R_B$** represents a hydrogen atom or a $(C_1$-$C_6)$alkylgroup optionally substituted with one or more groups chosen from i) -Z-C(Z')-Z"-Rc with Z, Z', and Z", which may be identical or different, representing an oxygen or sulfur atom, and $N(R_D)$, Rc and $R_D$, which may be identical or different, representing a hydrogen atom or a $(C_1$-$C_4)$alkyl group; preferably, Z, Z' and Z" represent a group $N(R_D)$ such as NH; ii) (hetero)aryl such as imidazolyl, indolyl, phenyl, optionally substituted especially with a hydroxyl group; iii) (di)$(C_1$-$C_4)$(alkyl)amino, iv) -C(Z')-Z"-$R_c$, with Z', Z" and $R_c$ as defined above, in particular Z' represents an oxygen atom, Z" represents an oxygen atom or a group $N(R_D)$ such as NH, and $R_c$ represents a hydrogen

atom; v) -Z‴-$R_c$, with Z‴ representing an oxygen, sulfur or selenium atom or an NH group and Rc is as defined previously; in particular, RB represents a ($C_1$-$C_6$)alkyl group optionally substituted with i) -Z-C(Z')-Z"-Rc, with Z, Z', and Z", which may be identical or different, representing an oxygen or sulfur atom, and N($R_D$), $R_C$ and $R_D$, which may be identical or different, representing a hydrogen atom or a ($C_1$-$C_4$)alkyl group; preferably Z, Z' and Z" represent a group N($R_D$) such as NH;

or $R_A$ and $R_B$ form, together with the nitrogen atom which bears $R_A$ and with the carbon atom which bears $R_B$, a saturated 5- or 6-membered, preferably 5-membered, heterocycle such as a pyrrolidine ring.

**15.** The process as claimed in any one of the preceding claims, in which the energy supply step 2) consists in exposing the keratin materials to artificial light radiation with a wavelength of between 360 and 600 nm, preferably between 375 and 550 nm, more preferentially between 400 and 480 nm; preferably, the artificial light radiation is generated using a device chosen from arc lamps such as xenon lamps and mercury lamps, fluorescent lamps, incandescent lamps such as halogen lamps, light-emitting diodes (LED), organic light-emitting diodes (OLED) and lasers.

**16.** A process for preparing composition (B'), which comprises the step of mixing i) one or more compounds of formula (I) as defined in any one of claims 1 to 7, ii) one or more polymerizable molecules chosen from those of formula **(II), (IIa), (IIb)** or **(IIc)** as defined in any one of claims 1 and 9 to 13 and optionally iii) one or more amines and/or one or more alcohols other than i) the compounds of formula **(I)**, and other than ii) the polymerizable molecules, preferably one or more amines preferably chosen from the amino acids as defined in claim 14; followed by a step of exposing said mixture to artificial or natural light radiation as defined in claim 1 or 15, in particular for at least 2 minutes, preferably for between 2 minutes up to 10 hours, more preferentially between 15 minutes and 5 hours, such as 3 hours.

**17.** A composition (B') obtained via the process as claimed in the preceding claim.

**18.** A multi-compartment device:

- comprising:

  * either in one compartment ingredient i) one or more compounds of formula **(I)** as defined in any one of claims 1 to 7, and in another compartment ingredient ii) one or more polymerizable molecules chosen from those of formula **(II), (IIa), (IIb)** or **(IIc)** as defined in any one of claims 1 and 9 to 13,
  * or in one compartment ingredients i) one or more compounds of formula **(I)** as defined in any one of claims 1 to 7, and ii) one or more polymerizable molecules chosen from those of formula **(II), (IIa), (IIb)** or **(IIc)** as defined in any one of claims 1 and 9 to 13, i) and ii) being together, and

- in another compartment an artificial-light-emitting device as defined in claim 15.

**19.** The cosmetic use i) of one or more flavin derivatives chosen from the compounds of formula (I) as defined in any one of claims 1 to 7, and ii) one or more polymerizable molecules chosen from those of formula **(II), (IIa), (IIb)** or **(IIc)** as defined in any one of claims 1 and 9 to 13, and artificial or natural light radiation as defined in claim 1 or 15; in particular for caring for and/or repairing keratin materials, especially human keratin materials such as the hair, preferably for repairing the hair.

## Patentansprüche

**1.** Verfahren zum Behandeln von Keratinmaterialien, insbesondere Keratinfasern, insbesondere menschlichen Keratinfasern wie dem Haar, umfassend:

1) einen Schritt des Aufbringens einer Zusammensetzung (A), umfassend:

i) ein oder mehrere Flavinderivate, ausgewählt aus den Verbindungen der nachstehenden Formel (I):

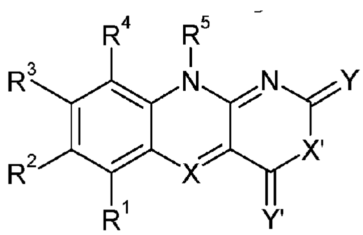

**(I)**

sowie den Salzen davon mit einer organischen oder mineralischen Säure oder Base, den optischen Isomeren, geometrischen Isomeren und Tautomeren davon und den Solvaten davon wie Hydraten;
wobei in Formel **(I):**

- **$R^1$, $R^2$, $R^3$** und **$R^4$,** die gleich oder verschieden sein können, für ein Wasserstoffatom, ein Halogenatom oder eine aus Hydroxyl, $(C_1-C_6)$Alkyl, $(C_1-C_6)$Alkoxy, $(C_1-C_6)$Alkylthio, Di $(C_1-C_6)$ (alkyl) amino, Nitro (so), insbesondere aus Wasserstoff und $(C_1-C_6)$Alkyl, ausgewählte Gruppe stehen; spezieller $R^1$ und $R^4$ für ein Wasserstoffatom stehen und $R^2$ und $R^3$ für ein Wasserstoffatom oder eine $(C_1-C_4)$Alkylgruppe wie Methyl stehen;
- **$R^5$** für ein Wasserstoffatom oder eine ($C_1$-Cs)Alkylgruppe, die gegebenenfalls durch eine oder mehrere Gruppen, die aus i) Hydroxyl, ii) $R^6$-C(Y")-O-, wobei $R^6$ für ein Wasserstoffatom oder eine $(C_1-C_4)$Alkyl- oder Aryl$(C_1-C_4)$alkylgruppe wie Benzyl steht und Y" für ein Sauerstoff- oder Schwefelatom oder $N(R^7)$ steht, wobei $R^7$ für ein Wasserstoffatom oder eine $(C_1-C_4)$Alkylgruppe steht, wobei Y" vorzugsweise für ein Sauerstoffatom steht, iii) Phosphorsäure $(HO)_2P(O)$-O-, iv) -O-$[(HO)P(O)-O]_n$-Suc-Het, wobei Suc für eine zweiwertige Zuckergruppe wie Ribose steht und Het für eine Heteroarylgruppe wie Adenin steht, n für eine ganze Zahl mit einem Wert von 0, 1 oder 2, vorzugsweise 2, steht; insbesondere die Gruppe Suc-Het für eine durch eine Adeningruppe substituierte Ribosegruppe steht, ausgewählt sind, substituiert ist, steht;
- **X** für ein Stickstoffatom oder eine Methylengruppe $C(R^8)$ steht, wobei $R^8$ für ein Wasserstoffatom oder eine $(C_1-C_4)$ Alkylgruppe steht; vorzugsweise X für ein Stickstoffatom steht;
- **X'** für ein Sauerstoffatom oder eine Gruppe $NR^9$ steht, wobei $R^9$ für ein Wasserstoffatom oder eine $(C_1-C_6)$Alkylgruppe steht; insbesondere X' für $NR^9$, vorzugsweise NH, steht;
- **Y** und **Y',** die gleich oder verschieden sein können, für ein Sauerstoff- oder Schwefelatom oder eine Gruppe $NR^{10}$ stehen, wobei $R^{10}$ für ein Wasserstoffatom oder eine $(C_1-C_6)$Alkylgruppe steht, vorzugsweise Y und Y' für ein Sauerstoffatom stehen; und

ii) ein oder mehrere polymerisierbare Moleküle; und

2) einen Schritt des Zuführens von Energie zu den Keratinmaterialien, der darin besteht, dass man die Materialien mindestens einer künstlichen oder natürlichen Lichtstrahlung aussetzt,
wobei es sich versteht, dass:

- Zusammensetzung (A) vor dem Aufbringen auf die Keratinmaterialien einen Schritt der Energiezufuhr durchlaufen kann, wobei der Schritt darin besteht, dass man die Zusammensetzung mindestens einer künstlichen oder natürlichen Lichtstrahlung aussetzt, was eine Zusammensetzung (B) ergibt, die dann auf die Materialien aufgetragen wird;
- die Schritte 1) und 2) gleichzeitig mit dem Aufbringen oder aufeinanderfolgend, vorzugsweise gleichzeitig mit dem Aufbringen, oder auch nach dem Aufbringen 1) und dann 2) durchgeführt werden können;
- das polymerisierbare Molekül bzw. die polymerisierbaren Moleküle mindestens eine ungesättigte Gruppe enthält bzw. enthalten, wobei:

  - es sich bei der ungesättigten Gruppe um -C≡CR' oder $-CR=C'_2$ handelt, wobei R und R', die gleich oder verschieden sein können, für ein Wasserstoffatom, eine $(C_1-C_4)$Alkylgruppe wie Methyl oder Carboxyl (COOH), vorzugsweise ein Wasserstoffatom oder eine $(C_1-C_4)$Alkylgruppe wie Methyl, stehen; **oder**
  - das polymerisierbare Molekül bzw. die polymerisierbaren Moleküle aus denjenigen der nachstehenden Formel **(II):**

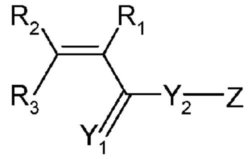

**(II)**

sowie den Salzen davon mit einer organischen oder mineralischen Säure oder Base, den optischen Isomeren, geometrischen Isomeren und Tautomeren und den Solvaten davon wie Hydraten ausgewählt ist bzw. sind;
wobei in Formel **(II):**

- **$R_1$** und **$R_2$**, die gleich oder verschieden sein können, für ein Wasserstoffatom oder eine $(C_1-C_8)$Alkyl-oder $(C_2-C_8)$Alkenylgruppe stehen, wobei die Alkyl- oder Alkenylgruppen

  - gegebenenfalls durch ein oder mehrere Heteroatome wie O, S und $N(R_5)$ unterbrochen sind, wobei $R_5$ für ein Wasserstoffatom oder eine $(C_1-C_4)$Alkylgruppe wie Methyl steht, und/oder
  - gegebenenfalls durch einen oder mehrere Reste, die aus den folgenden Resten ausgewählt sind: Hydroxyl, Di $(C_1-C_4)$ (alkyl) amino, $(C_1-C_4)$Alkoxy und $-C(Y'_1)-Y'_2-R'_4$ wie Carboxyl, substituiert sind;

- **$R_3$** wie für $R_2$ definiert ist oder auch für eine Gruppe $-C(Y'_1)-Y'_2-R'_4$ steht;
- **$Y_1, Y_2, Y'_1$** und **$Y'_2$**, die gleich oder verschieden sein können, für ein Sauerstoff- oder Schwefelatom oder $N(R_5)$ stehen, wobei $R_5$ für ein Wasserstoffatom oder eine $(C_1-C_4)$Alkylgruppe steht, vorzugsweise $Y_1$ und $Y'_1$ für ein Sauerstoffatom stehen und $Y_2$ und $Y'_2$ für ein Sauerstoffatom oder eine Gruppe $N(R_5)$ stehen; spezieller $Y_2$ und $Y'_2$ für ein Sauerstoffatom stehen; und
- **$R_4$** und **$R'_4$**, die gleich oder verschieden sein können, für ein Wasserstoffatom oder eine $(C_1-C_{16})$ - Alkylgruppe, die gegebenenfalls durch einen oder mehrere Reste, die gleich oder verschieden sein können und aus den folgenden Resten ausgewählt sind: a) Amino $(NH_2)$ , Mono- oder Di$(C_1-C_4)$alkylamino wie Dimethylamino, b) Hydroxyl (OH), c) $SO_3H$, d) Carboxyl $(CO_2H)$, e) Cyano, f) (Poly) (halogen) $(C_1-C_4)$ alkyl wie Trifluormethyl $(CF_3)$, g) $(C_1-C_4)$Alkoxycarbonyl, substituiert ist, stehen; wobei der Alkylrest gegebenenfalls durch ein oder mehrere Heteroatome wie O, S und $N(R_5)$ unterbrochen ist, wobei $R_5$ wie vorstehend definiert ist, und vorzugsweise $R_5$ für H oder Me steht;
- Z für einen Rest $R_4$ gemäß vorstehender Definition oder einen Rest **(Z')** der Formel:

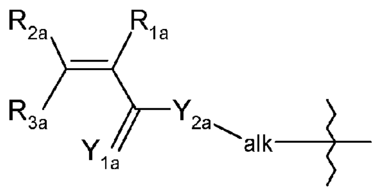

(**Z'**)

steht, wobei in der Formel **(Z'):**

$Y_{1a}$, Y2a, $R_{1a}$, R2a und R3a jeweils und unabhängig die gleiche Bedeutung wie vorstehend definiertes Y1, $Y_2$, $R_1$, $R_2$ und $R_3$ haben;
alk einen zweiwertigen $(C_1-C_{16})$ Alkylenrest, der gegebenenfalls durch ein oder mehrere Sauerstoff- oder Schwefelatome oder durch eine oder mehrere NH-Gruppen unterbrochen ist, bedeutet, wobei der Alkylenrest gegebenenfalls durch einen oder mehrere Reste, die aus Hydroxyl und einem Rest der Formel (Z"):

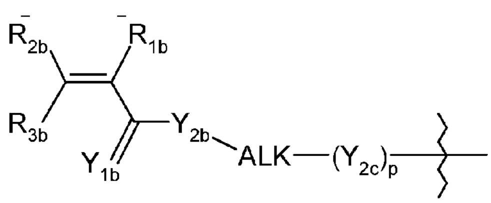

(Z")

ausgewählt sind, substituiert ist,
wobei in der Formel **(Z"):**

- $Y_{1b}$, $Y_{2b}$, $Y_{2c}$, $R_{1b}$, $R_{2b}$ und $R_{3b}$ jeweils und unabhängig die gleiche Bedeutung wie vorstehend definiertes $Y_1$, $Y_2$, $R_1$, $R_2$ und $R_3$ haben, ALK einen ($C_1$-$C_6$) Alkylenrest (zweiwertigen Rest) wie Methylen oder Propylen bedeutet und p gleich 0 oder 1 ist;





2. Verfahren nach dem vorhergehenden Anspruch, wobei die Verbindung bzw. die Verbindungen der Formel (I) so beschaffen ist bzw. sind, dass $\mathbf{R^1}$, $\mathbf{R^2}$, $\mathbf{R^3}$ und $\mathbf{R^4}$, die gleich oder verschieden sein können, für ein Wasserstoffatom, ($C_1$-$C_6$)Alkyl, ($C_1$-$C_6$)Alkoxy oder Di($C_1$-$C_4$)(alkyl)amino stehen, insbesondere aus Wasserstoff und ($C_1$-$C_4$)Alkyl ausgewählt sind; vorzugsweise $R^1$ und $R^4$ für ein Wasserstoffatom stehen und $R^2$ und $R^3$ für ein Wasserstoffatom oder eine ($C_1$-$C_4$)Alkylgruppe wie Methyl, vorzugsweise eine ($C_1$-$C_4$)Alkylgruppe wie Methyl, stehen.

3. Verfahren nach einem der vorhergehenden Ansprüche, wobei die Verbindung bzw. die Verbindungen der Formel **(I)** so beschaffen ist bzw. sind, dass $R^5$ für ein Wasserstoffatom oder eine ($C_1$-$C_6$)Alkylgruppe wie Methyl steht.

4. Verfahren nach einem der Ansprüche 1 und 2, wobei die Verbindung bzw. die Verbindungen der Formel **(I)** so beschaffen ist bzw. sind, dass $\mathbf{R_5}$ für eine ($C_1$-$C_6$)Alkylgruppe, die durch eine oder mehrere Gruppen, die gleich oder verschieden sein können und aus i) Hydroxyl, ii) $R^6$-C(O)-O-, wobei $R^6$ für ein Wasserstoffatom oder eine ($C_1$-$C_4$)Alkylgruppe wie Methyl oder n-Propyl steht, iii) Phosphorsäure $(HO)_2P(O)$-O-, iv) -O-[(HO)P(O)-O]$_n$-Suc-Het, wobei Suc für ein Monosaccharid wie Ribose steht und Het für eine gegebenenfalls substituierte Heteroarylgruppe, insbesondere gegebenenfalls substituiertes Pyrimidyldion wie Adenin, steht, n für eine ganze Zahl mit einem Wert von 1 oder 2, vorzugsweise 2, steht; insbesondere Suc-Het für eine durch eine Adeningruppe gegebenenfalls substituierte Ribosegruppe, vorzugsweise $\alpha$-D-Ribose, steht, ausgewählt sind, substituiert ist, steht.

5. Verfahren nach einem der vorhergehenden Ansprüche, wobei die Verbindung bzw. die Verbindungen der Formel **(I)** so beschaffen ist bzw. sind, dass, zusammengenommen oder separat, **X** für ein Stickstoffatom steht; **X**' für eine Gruppe $NR^9$, wobei $R^9$ für ein Wasserstoffatom oder eine ($C_1$-$C_6$)Alkylgruppe steht; insbesondere $NR^9$ vorzugsweise NH, steht; und **Y** und **Y'** gleich sind und spezieller für ein Sauerstoff- oder Schwefelatom stehen; vorzugsweise Y und Y' für ein Sauerstoffatom stehen.

6. Verfahren nach einem der vorhergehenden Ansprüche, wobei die Verbindung bzw. die Verbindungen der Formel **(I)** aus den nachstehenden Verbindungen **1** bis **7**:

| | |
|---|---|
| Struktur<br>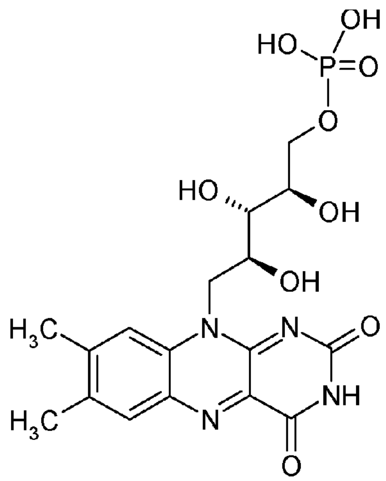<br>(2*R*,3*S*,4*S*)-5-(7,8-Dimethyl-2,4-dioxo-3,4-dihydrobenzo[g]pteridin-10(2H)-yl)-2,3,4-trihydroxypentyldihydrogenphosphat oder Riboflavin-5'-phosphat | 1 |
| 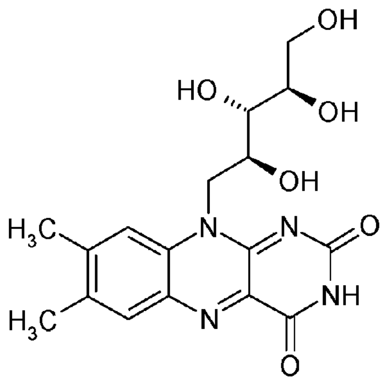<br>7,8-Dimethyl-10-((2*R*,3*R*,4*S*)-2,3,4,5-tetrahydroxypentyl)benzo[g]pteridin-2,4-(3H,10H)-dion oder Vitamin $B_2$, Lactoflavin | 2 |

(fortgesetzt)

| Struktur | |
|---|---|
| 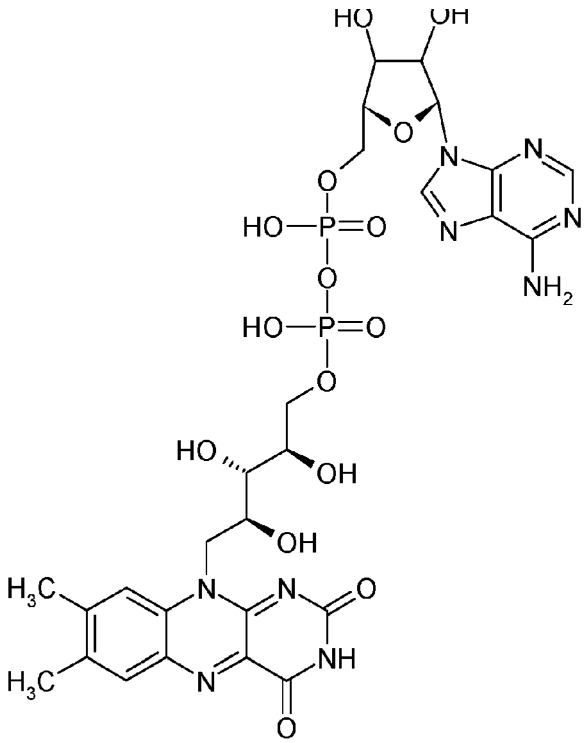<br>Flavinadenindinucleotid (FAD) | 3 |
| 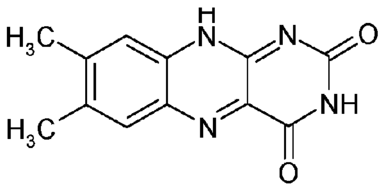<br>7,8-Dimethylbenzo[g]pteridin-2,4(3H,10H)-dion | 4 |
| 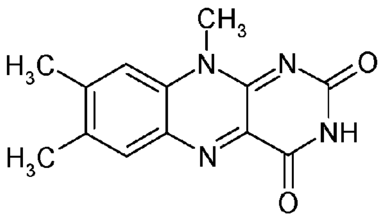<br>7, 8, 10-Trimethylbenzo[g]pteridin-2,4 (3H,10H)-dion | 5 |

(fortgesetzt)

| Struktur | |
|---|---|
| 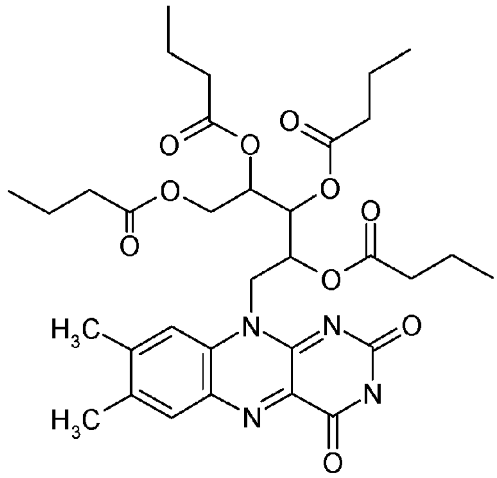 Riboflavintetrabutyrat | 6 |
| 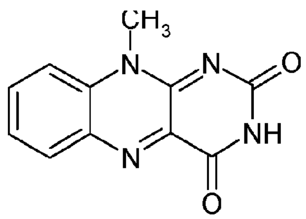 10-Methylbenzo[g]pteridin-2,4(3H,10H)-dion | 7 |

sowie den Salzen davon mit einer organischen oder mineralischen Säure oder Base, den optischen Isomeren und Tautomeren davon und den Solvaten davon wie Hydraten, insbesondere dem Salz davon mit einem Alkalimetall wie Natrium, ausgewählt ist bzw. sind.

**7.** Verfahren nach einem der vorhergehenden Ansprüche, wobei die Verbindung bzw. die Verbindungen der Formel **(I)** in einer Menge inklusive zwischen 0,01 und 30 Gew.-%, bezogen auf das Gesamtgewicht der Zusammensetzung (A), (B) bzw. (B'), insbesondere zwischen 0,05 und 20 Gew.-%, spezieller zwischen 0,1 und 10 Gew.-%, vorzugsweise zwischen 1 und 5 Gew.-%, bezogen auf das Gesamtgewicht der Zusammensetzung (A) bzw. (B), vorliegt bzw. vorliegen.

**8.** Verfahren nach einem der vorhergehenden Ansprüche, wobei ii) das polymerisierbare Molekül bzw. die polymerisierbaren Moleküle ein Molekulargewicht von kleiner als oder gleich 500 g/mol, vorzugsweise ein Molekulargewicht inklusive zwischen 26 und 400 g/mol, aufweisen.

**9.** Verfahren nach einem der vorhergehenden Ansprüche, wobei $\mathbf{R_1}$ für ein Wasserstoffatom, eine $(C_1\text{-}C_4)$Alkylgruppe wie Methyl oder eine $(C_1\text{-}C_4)$Alkylgruppe, die durch einen aus Hydroxyl und $-C(Y'_1)-Y'_2-R'_4$, insbesondere $-C(O)-O-R'_4$, wobei $R'_4$ wie im vorhergehenden Anspruch definiert ist, substituiert ist, steht; vorzugsweise $\mathbf{R_1}$ für eine $(C_1\text{-}C_4)$Alkylgruppe, die durch eine Carboxylgruppe substituiert ist, wie Carboxymethyl steht.

**10.** Verfahren nach einem der vorhergehenden Ansprüche, wobei das polymerisierbare Molekül bzw. die polymerisierbaren Moleküle aus denjenigen der nachstehenden Formel **(IIa):**

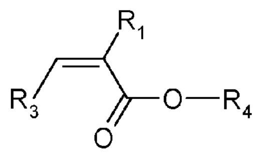

sowie den Salzen davon mit einer organischen oder mineralischen Säure oder Base, den optischen Isomeren, geometrischen Isomeren und Tautomeren und den Solvaten davon wie Hydraten ausgewählt ist bzw. sind; wobei in Formel **(IIa)** $\mathbf{R_1}$**,** $\mathbf{R_3}$ und $\mathbf{R_4}$ wie in den Ansprüchen 5 bis 9 definiert sind; vorzugsweise das polymerisierbare Molekül bzw. die polymerisierbaren Moleküle aus (($C_1$-$C_4$)Alkyl)acrylsäure wie Methacrylsäure, Acrylsäure oder Itaconsäure ausgewählt ist bzw. sind.

**11.** Verfahren nach einem der vorhergehenden Ansprüche, wobei das polymerisierbare Molekül bzw. die polymerisierbaren Moleküle aus denjenigen der nachstehenden Formel **(IIb):**

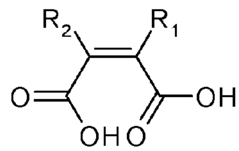

**(IIb)**

sowie den Salzen davon mit einer organischen oder mineralischen Säure oder Base, den optischen Isomeren, geometrischen Isomeren und Tautomeren und den Solvaten davon wie Hydraten ausgewählt ist bzw. sind; wobei in Formel **(IIb)** $\mathbf{R_1}$ und $\mathbf{R_2}$ wie in den Ansprüchen 5 bis 10 definiert sind; insbesondere aus Wasserstoff und ($C_1$-$C_4$)Alkyl wie Methyl, vorzugsweise Wasserstoff, ausgewählt sind.

**12.** Verfahren nach einem der vorhergehenden Ansprüche, wobei das polymerisierbare Molekül bzw. die polymerisierbaren Moleküle aus denjenigen der nachstehenden Formel (IIc):

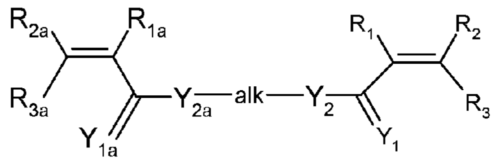

**(IIc)**

sowie den Salzen davon mit einer organischen oder mineralischen Säure oder Base, den optischen Isomeren, geometrischen Isomeren und Tautomeren und den Solvaten davon wie Hydraten ausgewählt ist bzw. sind; wobei in Formel **(IIc)** $\mathbf{R_1}$**,** $\mathbf{R_2}$**,** $R_3$, $Y_1$, $Y_2$, alk, $Y_{2a}$, $Y_{1a}$, $R_{1a}$, $R_{2a}$ und $R_{3a}$ wie in den Ansprüchen 5 bis 11 definiert sind; insbesondere derart, dass $R_1 = R_{1a}$ und $R_2 = R_{2a}$ und $R_3 = R_{3a}$ und $Y_1 = Y_{1a}$ und $Y_2 = Y_{2a}$; vorzugsweise aus den nachstehenden Verbindungen (a) bis (d) ausgewählt ist bzw. sind:

| | |
|---|---|
| 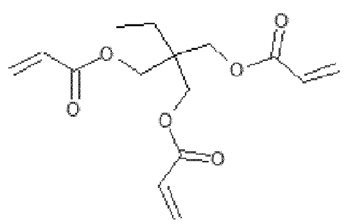 | 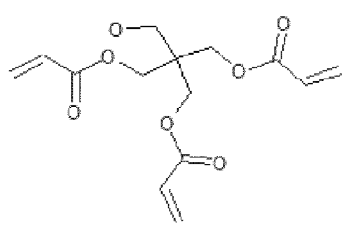 |
| **(a)** | **(b)** |

(fortgesetzt)

| | |
|---|---|
| 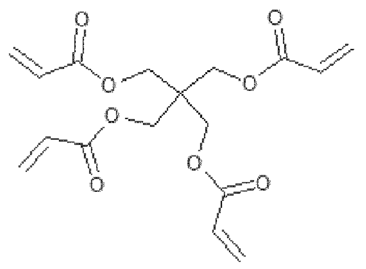 | 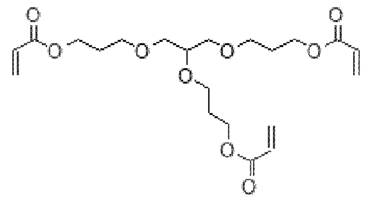 |
| **(c)** | **(d)** |

**13.** Verfahren nach einem der vorhergehenden Ansprüche, wobei das polymerisierbare Molekül bzw. die polymerisierbaren Moleküle in einer Menge inklusive zwischen 0,01 und 50 Gew.-%, bezogen auf das Gesamtgewicht der Zusammensetzungen (A) bzw. (B), insbesondere zwischen 0,1 und 40 Gew.-%, spezieller zwischen 1 und 30 Gew.-%, vorzugsweise zwischen 2 und 20 Gew.-%, weiter bevorzugt zwischen 5 und 10 Gew.-%, bezogen auf das Gesamtgewicht der Zusammensetzung (A) bzw. (B), vorliegt bzw. vorliegen.

**14.** Verfahren nach einem der vorhergehenden Ansprüche, wobei Zusammensetzung (A) bzw. (B) ein oder mehrere Amine, die von i) den Riboflavinen der Formel **(I)** verschieden sind und von ii) den polymerisierbaren Molekülen verschieden sind, umfasst; wobei die Amine aromatisch oder nichtaromatisch, gesättigt oder ungesättigt, oligomer oder polymer und natürlich oder synthetisch sein können; es sich bei dem bzw. den Aminen vorzugsweise um Aminosäuren handelt, die aus den Verbindungen der nachstehenden Formel **(III):**

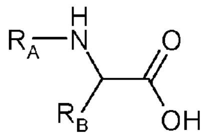

**(III)**

sowie den Salzen davon mit einer organischen oder mineralischen Säure oder Base, den optischen Isomeren, geometrischen Isomeren und Tautomeren davon und den Solvaten davon wie Hydraten ausgewählt sind;

• **$R_A$** für ein Wasserstoffatom, eine $(C_1\text{-}C_4)$Alkyl-oder $(C_1\text{-}C_4)$Alkylcarbonylgruppe steht oder auch zusammen mit dem Rest $R_B$ und dem Stickstoff- und Kohlenstoffatom, die sie jeweils tragen, einen 5- bis 10-gliedrigen monocyclischen oder bicyclischen Heterocyclus wie Pyrrolidinyl bildet; vorzugsweise $R_A$ für ein Wasserstoffatom steht;
• **$R_B$** für ein Wasserstoffatom oder eine $(C_1\text{-}C_6)$ - Alkylgruppe, die gegebenenfalls durch eine oder mehrere Gruppen, die aus i) Z-C(Z')-Z"-$R_c$, wobei Z, Z' und Z", die gleich oder verschieden sein können, für ein Sauerstoff- oder Schwefelatom und N($R_D$) stehen, wobei $R_c$ und $R_D$, die gleich oder verschieden sein können, für ein Wasserstoffatom oder eine $(C_1\text{-}C_4)$Alkylgruppe stehen;

vorzugsweise Z, Z' und Z" für eine Gruppe N($R_D$) wie NH stehen; ii) (Hetero)aryl wie Imidazolyl, Indolyl, Phenyl, das gegebenenfalls insbesondere durch eine Hydroxylgruppe substituiert ist; iii) (Di)$(C_1\text{-}C_4)$ (alkyl) amino, iv) -C (Z') -Z" -Rc, wobei Z', Z" und Rc wie oben definiert sind, insbesondere Z' für ein Sauerstoffatom steht, Z" für ein Sauerstoffatom oder eine Gruppe N($R_D$) wie NH steht und Rc für ein Wasserstoffatom steht; v) -Z'''-$R_c$, wobei Z''' für ein Sauerstoff-, Schwefel- oder Selenatom oder eine NH-Gruppe steht und $R_c$ wie oben definiert ist, ausgewählt sind, substituiert ist, steht; insbesondere $R_B$ für eine $(C_1\text{-}C_6)$Alkylgruppe, die gegebenenfalls durch i) -Z-C(Z')-Z"-Rc, wobei Z, Z' und Z", die gleich oder verschieden sein können, für ein Sauerstoff- oder Schwefelatom und N($R_D$) stehen, wobei $R_c$ und $R_D$, die gleich oder verschieden sein können, für ein Wasserstoffatom oder eine $(C_1\text{-}C_4)$ Alkylgruppe stehen; vorzugsweise Z, Z' und Z" für eine Gruppe N($R_D$) wie NH stehen; substituiert ist, steht;
oder $R_A$ und $R_B$ zusammen mit dem Stickstoffatom, das $R_A$ trägt, und mit dem Kohlenstoffatom, das $R_B$ trägt, einen gesättigten 5- oder 6-gliedrigen, vorzugsweise 5-gliedrigen, Heterocyclus wie einen Pyrrolidinring bilden.

**15.** Verfahren nach einem der vorhergehenden Ansprüche, wobei der Energiezuführungsschritt 2) darin besteht, dass man die Keratinmaterialien künstlicher Lichtstrahlung mit einer Wellenlänge zwischen 360 und 600 nm, vorzugsweise zwischen 375 und 550 nm, weiter bevorzugt zwischen 400 und 480 nm, aussetzt; vorzugsweise die künstliche Lichtstrahlung unter Verwendung einer aus Bogenlampen wie Xenonlampen und Quecksilberlampen, Fluoreszenzlampen, Glühlampen wie Halogenlampen, Leuchtdioden (LED), organischen Leuchtdioden (OLED) und Lasern ausgewählten Vorrichtung erzeugt wird.

**16.** Verfahren zur Herstellung von Zusammensetzung (B'), das den Schritt des Mischens von i) einer oder mehreren Verbindungen der Formel **(I)** gemäß einem der Ansprüche 1 bis 7, ii) einem oder mehreren polymerisierbaren Molekülen, ausgewählt aus denjenigen der Formel **(II), (IIa), (IIb)** oder **(IIc)** gemäß einem der Ansprüche 1 und 9 bis 13, und gegebenenfalls (iii) einem oder mehreren Aminen und/oder einem oder mehreren Alkoholen, die von (i) den Verbindungen der Formel **(I)** verschieden sind und von ii) den polymerisierbaren Molekülen verschieden sind, vorzugsweise einem oder mehreren Aminen, vorzugsweise ausgewählt aus den Aminosäuren gemäß Anspruch 14; gefolgt von einem Schritt, bei dem man die Mischung künstlicher oder natürlicher Lichtstrahlung gemäß Anspruch 1 oder 15 aussetzt, insbesondere über einen Zeitraum von mindestens 2 Minuten, vorzugsweise über einen Zeitraum zwischen 2 Minuten bis zu 10 Stunden, weiter bevorzugt zwischen 15 Minuten und 5 Stunden, wie 3 Stunden, umfasst.

**17.** Zusammensetzung (B'), erhalten gemäß dem Verfahren nach dem vorhergehenden Anspruch.

**18.** Vorrichtung mit mehreren Kompartimenten:

- umfassend:

  * entweder in einem Kompartiment Bestandteil i) eine oder mehrere Verbindungen der Formel **(I)** gemäß einem der Ansprüche 1 bis 7 und in einem anderen Kompartiment Bestandteil ii) ein oder mehrere polymerisierbare Moleküle, ausgewählt aus denjenigen der Formel **(II), (IIa), (IIb)** oder **(IIc)** gemäß einem der Ansprüche 1 und 9 bis 13,
  * oder in einem Kompartiment Bestandteile i) eine oder mehrere Verbindungen der Formel **(I)** gemäß einem der Ansprüche 1 bis 7 und ii) ein oder mehrere polymerisierbare Moleküle, ausgewählt aus denjenigen der Formel **(II), (IIa), (IIb)** oder **(IIc)** gemäß einem der Ansprüche 1 und 9 bis 13, wobei i) und ii) zusammen vorliegen, und

- in einem anderen Kompartiment eine künstliches Licht emittierende Vorrichtung gemäß Anspruch 15.

**19.** Kosmetische Verwendung von i) einem oder mehreren Flavinderivaten, ausgewählt aus den Verbindungen der Formel **(I)** gemäß einem der Ansprüche 1 bis 7, und ii) einem oder mehreren polymerisierbaren Molekülen, ausgewählt aus denjenigen der Formel **(II), (IIa), (IIb)** oder **(IIc)** gemäß einem der Ansprüche 1 und 9 bis 13 und künstliche oder natürliche Lichtstrahlung gemäß Anspruch 1 oder 15; insbesondere zum Pflegen und/oder Reparieren von Keratinmaterialien, insbesondere menschlichen Keratinmaterialien wie dem Haar, vorzugsweise zum Reparieren des Haars.

## Revendications

**1.** Procédé pour le traitement de matières kératiniques, en particulier de fibres kératiniques, notamment de fibres kératiniques humaines telles que les cheveux, comprenant :

1) une étape d'application d'une composition (A) comprenant :

i) un ou plusieurs dérivés de flavine choisis parmi les composés de formule **(I)** ci-dessous :

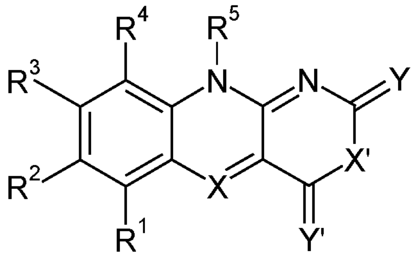

**(I)**

et également leurs sels d'acide ou de base, organique ou minéral(e), leurs isomères optiques, leurs isomères géométriques et leurs formes tautomères, et leurs solvates tels que les hydrates ;
dans laquelle formule **(I) :**

- **$R^1$, $R^2$, $R^3$** et **$R^4$**, qui peuvent être identiques ou différents, représentent un atome d'hydrogène, un atome d'halogène ou un groupe choisi parmi hydroxyle, $(C_1-C_6)$alkyle, $(C_1-C_6)$alcoxy, $(C_1-C_6)$ alkylthio, (di) $(C_1-C_6)$(alkyl) amino, nitro (so), en particulier choisi parmi hydrogène et $(C_1-C_6)$alkyle; plus particulièrement $R^1$ et $R^4$ représentent un atome d'hydrogène, et $R^2$ et $R^3$ représentent un atome d'hydrogène ou un groupe $(C_1-C_4)$ alkyle tel que méthyle ;
- **$R^5$** représente un atome d'hydrogène ou un groupe $(C_1-C_8)$ alkyle éventuellement substitué par un ou plusieurs groupes choisis parmi i) hydroxyle, ii) $R^6$-C(Y")-O-, $R^6$ représentant un atome d'hydrogène ou un groupe $(C_1-C_4)$ alkyle ou aryl $(C_1-C_4)$ alkyle tel que benzyle et Y" représente un atome d'oxygène ou de soufre, ou N($R^7$), $R^7$ représentant un atome d'hydrogène ou un groupe $(C_1-C_4)$ alkyle, préférablement Y" représentant un atome d'oxygène,

iii) (HO) $_2$P(O)-O- phosphorique, iv) -O-[(HO)P(O)-O]$_n$-Suc-Het avec Suc représentant un groupe sucre divalent tel que ribose, et Het représentant un groupe hétéroaryle tel qu'adénine, n un entier égal à 0, 1, ou 2, préférablement 2 ; en particulier le groupe Suc-Het représente un groupe ribose substitué par un groupe adénine ;

- **X** représente un atome d'azote ou un groupe méthylène C($R^8$), $R^8$ représentant un atome d'hydrogène ou un groupe $(C_1-C_4)$alkyle ; préférablement X représente un atome d'azote ;
- **X**' représente un atome d'oxygène ou un groupe $NR^9$, $R^9$ représentant un atome d'hydrogène ou un groupe $(C_1-C_6)$ alkyle ; en particulier X' désigne $NR^9$, préférablement NH ;
- **Y** et **Y**', qui peuvent être identiques ou différents, représentent un atome d'oxygène ou de soufre ou un groupe $NR^{10}$, $R^{10}$ représentant un atome d'hydrogène ou un groupe $(C_1-C_6)$alkyle, préférablement Y et Y' représentent un atome d'oxygène ; et

ii) une ou plusieurs molécules polymérisables ; et

2) une étape d'apport énergétique auxdites matières kératiniques, qui consiste à exposer lesdites matières à au moins un rayonnement lumineux artificiel ou naturel,
étant entendu que :

- la composition (A) peut subir une étape d'apport énergétique avant d'être appliquée aux matières kératiques, consitant à exposer ladite composition à au moins un rayonnement lumineux artificiel ou naturel pour conduire à une composition (B) qui sera ensuite appliquée auxdites matières ;
- les étapes 1) et 2) pouvent être réalisées simultanément avec l'application, ou séquentiellement, préférablement simultanément avec l'application ou alors séquentiellement avec l'application 1) et puis 2) ;
- la ou les molécules polymérisables comprenant au moins un groupe insaturé :

  - le groupe insaturé étant -C≡CR' ou -CR=CR'$_2$, R et R', qui peuvent être identiques ou différents, représentant un atome d'hydrogène, un groupe $(C_1-C_4)$ alkyle tel que méthyle ou carboxyle (COOH), préférablement un atome d'hydrogène ou un groupe $(C_1-C_4)$ alkyle tel que méthyle ; **ou**
  - la ou les molécules polymérisables sont choisies parmi celles de formule **(II)** ci-dessous :

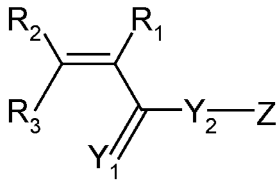

(II)

et également leurs sels d'acide ou de base, organique ou minéral(e), leurs isomères optiques, leurs isomères géométriques et leurs formes tautomères, et leurs solvates tels que les hydrates ;
dans laquelle formule **(II) :**

- $R_1$, et $R_2$, qui peuvent être identiques ou différents, représentent un atome d'hydrogène, un groupe ($C_1$-$C_8$) alkyle ou ($C_2$-$C_8$) alcényle, lesdits groupes alkyle ou alcényle étant :

  - éventuellement interrompus par un ou plusieurs hétéroatomes tels que O, S et N(Rs), $R_5$ représentant un atome d'hydrogène ou un groupe ($C_1$-$C_4$) alkyle tel que méthyle, et/ou
  - éventuellement substitués par un ou plusieurs radicaux choisis parmi les radicaux suivants : hydroxyle, (di) ($C_1$-$C_4$) (alkyl) amino, ($C_1$-$C_4$) alcoxy et -C(Y'$_1$)-Y'$_2$-R'$_4$ tel que carboxyle ;

- $R_3$ est tel que défini pour $R_2$, ou alors représente un groupe -C(Y'$_1$)-Y'$_2$-R'$_4$ ;
- **$Y_1$, $Y_2$, Y'$_1$,** et **Y'$_2$**, qui peuvent être identiques ou différents, représentent un atome d'oxygène ou de soufre ou N($R_5$) , $R_5$ représentant un atome d'hydrogène ou un groupe ($C_1$-$C_4$) alkyle, préférablement $Y_1$ et Y'$_1$ représentent un atome d'oxygène et $Y_2$ et Y'$_2$, représentent un atome d'oxygène ou un groupe N($R_5$) ; plus particulièrement $Y_2$ et Y'$_2$ représentent un atome d'oxygène ; et
- **$R_4$** et **R'$_4$**, qui peuvent être identiques ou différents, représentent un atome d'hydrogène ou un groupe ($C_1$-$C_{16}$) alkyle éventuellement subsitué par un ou plusieurs radicaux, qui peuvent être identiques ou différents, choisis parmi a) amino ($NH_2$) , monoalkylamino ou dialkylamino en $C_1$-$C_4$ tel que diméthylamino, b) hydroxyle (OH), c) $SO_3H$, d) carboxy ($CO_2H$), e) cyano, f) (poly) (halogéno) ($C_1$-$C_4$) alkyle tel que trifluorométhyle ($CF_3$), g) ($C_1$-$C_4$)alcoxycarbonyle ; ledit radical alkyle étant éventuellement interrompu par un ou plusieurs hétéroatomes, tels que O, S et N($R_5$) avec $R_5$ tel que défini précédemment et préférablement $R_5$ désignant H ou Me ;
- Z désigne un radical $R_4$ tel que défini précédemment ou un radical **(Z')** de formule :

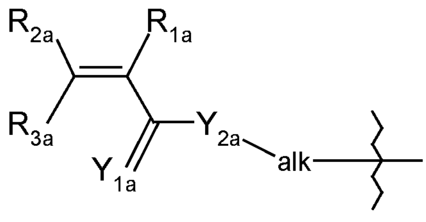

(Z')

dans laquelle formule **(Z') :**

  - $Y_{1a}$, $Y_{2a}$, $R_{1a}$, $R_{2a}$ et $R_{3a}$ ont, respectivement et indépendamment, la même signification que $Y_1$, $Y_2$, $R_1$, $R_2$ et $R_3$ définis précédemment ;
  - alk désigne un radical divalent ($C_1$-$C_{16}$) alkylène éventuellement intérrompu par un ou plusieurs atomes d'oxygène ou de soufre ou par un ou plusieurs groupes NH, ledit radical alkylène étant éventuellement substitué par un ou plusieurs radicaux choisis parmi hydroxyle et un radical de formule (Z") :

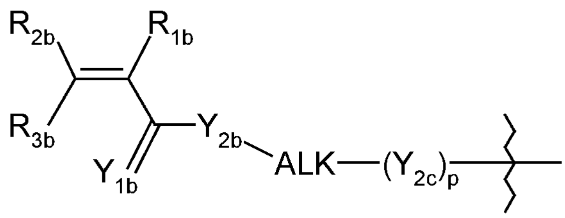

(Z′′)

dans laquelle formule (Z') :

- $Y_{1b}$, $Y_{2b}$, $Y_{2c}$, $R_{1b}$, $R_{2b}$ et $R_{3b}$ ont, respectivement et indépendamment, la même signification que $Y_1$, $Y_2$, $R_1$, $R_2$ et $R_3$ définis précédemment, ALK désignant un radical alkylène (radical divalent) en ($C_1$-$C_6$) tel que méthylène ou propylène et p étant égal à 0 ou 1 ;

- désignant le point de fixation entre alk et $Y_2$ dans le cas de la formule (Z') et désignant le point de fixation entre ($Y_2$)p et alk dans le cas de la formule (Z").

2. Procédé selon la revendication précédente, dans lequel le ou les composés de formule **(I)** sont tels que $\mathbf{R^1}$**,** $\mathbf{R^2}$**,** $\mathbf{R^3}$ et $\mathbf{R^4}$, qui peuvent être identiques ou différents, représentent un atome d'hydrogène, ($C_1$-$C_6$) alkyle, ($C_1$-$C_6$) alcoxy ou (di) ($C_1$-$C_4$) (alkyl) amino, en particulier choisis parmi hydrogène et ($C_1$-$C_4$) alkyle ; préférablement $R^1$ et $R^4$ représentent un atome d'hydrogène, et $R^2$ et $R^3$ représentent un atome d'hydrogène ou un groupe ($C_1$-$C_4$) alkyle tel que méthyle, préférablement un groupe ($C_1$-$C_4$)alkyle tel que méthyle.

3. Procédé selon l'une ou l'autre des revendications précédentes, dans lequel le ou les composés de formule **(I)** sont tels que $R^5$ représente un atome d'hydrogène ou un groupe ($C_1$-$C_6$)alkyle tel que méthyle.

4. Procédé selon l'une ou l'autre des revendications 1 ou 2, dans lequel le ou les composés de formule **(I)** sont tels que $\mathbf{R_5}$ représente un groupe ($C_1$-$C_6$) alkyle substitué par un ou plusieurs groupes, identiques ou différents, choisis parmi i) hydroxyle, ii) $R^6$-C(O)-O-avec $R^6$ représentant un atome d'hydrogène, un groupe ($C_1$-$C_4$) alkyle tel que méthyle ou n-propyle ; iii) $(HO)_2P(O)$-O- phosphorique, iv) -O-$[(HO)P(O)-O]_n$-Suc-Het avec Suc représentant un monosaccharide tel que le ribose, et Het représentant un groupe hétéroaryle éventuellement substitué, en particulier pyrimidyle-dione éventuellement substitué tel que adénine, n un entier vallant 1 ou 2, préférablement 2 ; en particulier Suc-Het représente un groupe ribose, préférablement $\alpha$-D-ribose, éventuellement substitué par un groupe adénine.

5. Procédé selon l'une quelconque des revendications précédentes, dans lequel le ou les composés de formule **(I)** sont tels que, pris ensemble ou séparément, **X** représente un atome d'azote ; **X**' représente un groupe $NR^9$ avec $R^9$ représentant un atome d'hydrogène ou un groupe ($C_1$-$C_6$)alkyle, en particulier $NR^9$ préférablement NH ; et **Y** et **Y**' sont identiques, plus particulièrement représentent un atome d'oxygène ou de soufre, préférablement Y et Y' représentent un atome d'oxygène.

6. Procédé selon l'une quelconque des revendications précédentes, dans lequel le ou les composés de formule **(I)** sont choisis parmi les composés **1** à **7** ci-dessous :

| Structure | |
|---|---|
| 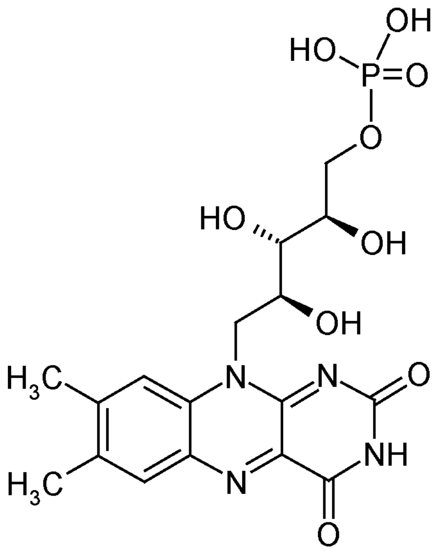<br>dihydrogénophosphate de (2*R*,3*S*,4*S*)-5-(7,8-diméthyl-2,4-dioxo-3,4-dihydrobenzo[g]ptéridin-10(2H)-yl)-2,3,4-trihydroxypentyle ou 5'-phosphate de riboflavine | 1 |
| 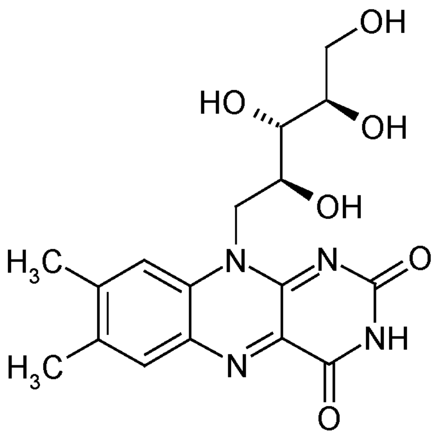<br>7,8-diméthyl-10-((2*R*,3*R*,4*S*)-2,3,4,5-tétrahydroxypentyl)benzo[g]ptéridine-2,4-(3H,10H)-dione, ou vitamine $B_2$, lactoflavine | 2 |

(suite)

| Structure | |
|---|---|
| 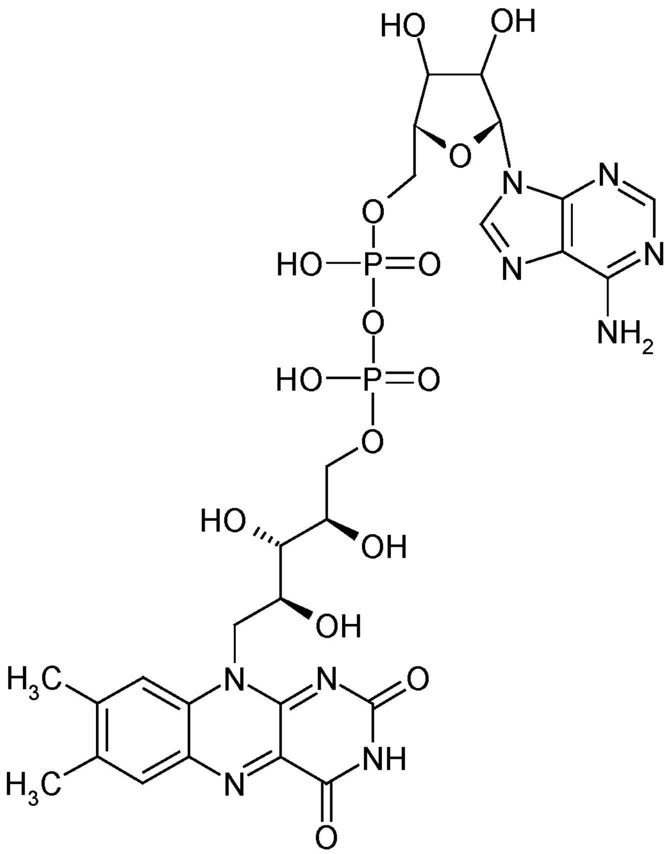 Flavine adénine dinucléotide (FAD) | 3 |
| 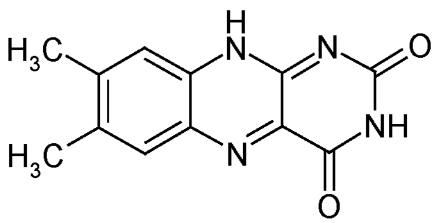 7,8-diméthylbenzo[g]ptéridine-2,4(3H,10H)-dione | 4 |
| 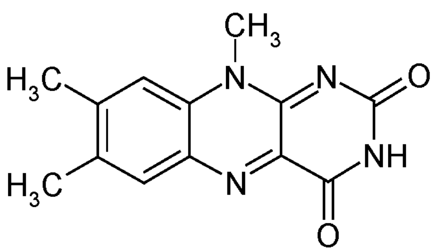 7,8,10-triméthylbenzo[g]ptéridine-2,4(3H,10H)-dione | 5 |

(suite)

| Structure | |
|---|---|
| 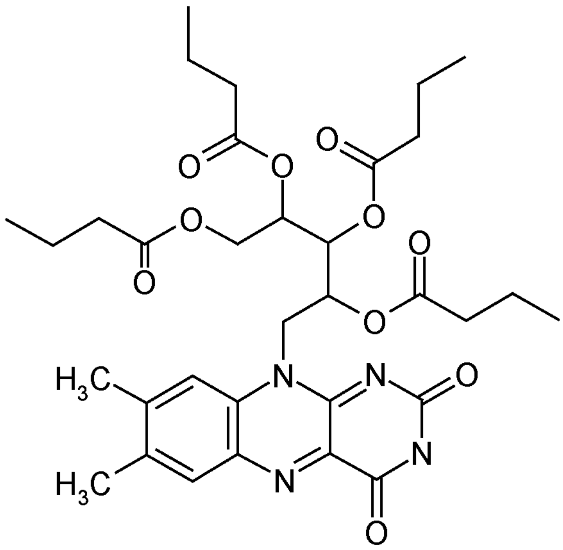 Tétrabutyrate de riboflavine | 6 |
| 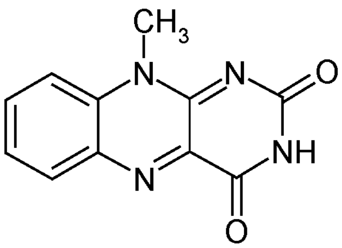 10-méthylbenzo[g]ptéridine-2,4(3H,10H)-dione | 7 |

et également leurs sels d'acide ou de base, organique ou minéral(e), leurs isomères optiques et leurs formes tautomères, et leurs solvates tels que les hydrates, en particulier leur sel avec un métal alcalin tel que le sodium.

**7.** Procédé selon l'une quelconque des revendications précédentes, dans lequel le ou les composés de formule **(I)** se trouvent en une quantité comprise inclusivement entre 0,01 % et 30 % en poids par rapport au poids total de la composition (A), (B) ou (B'), en particulier entre 0,05 % et 20 %, plus particulièrement entre 0,1 % et 10 %, préférentiellement entre 1 % et 5 % en poids par rapport au poids total de la composition (A) ou (B).

**8.** Procédé selon l'une quelconque des revendications précédentes, dans lequel ii) la ou les molécules polymérisables possèdent un poids moléculaire inférieur ou égal à 500 g/mole, préférablement un poids moléculaire compris inclusivement entre 26 et 400 g/mole.

**9.** Procédé selon l'une quelconque des revendications précédentes, dans lequel $\mathbf{R_1}$ représente un atome d'hydrogène, un groupe ($C_1$-$C_4$) alkyle tel que méthyle ou un groupe ($C_1$-$C_4$)alkyle substitué par un radical choisi parmi hydroxyle et -C(Y'$_1$)-Y'$_2$-R'$_4$, en particulier - C(O)-O-R'$_4$, R'$_4$ étant tel que défini dans la revendication précédente ; préférablement $\mathbf{R_1}$ étant un groupe ($C_1$-$C_4$)alkyle susbtitué par un groupe carboxyle tel que carboxyméthyle.

**10.** Procédé selon l'une quelconque des revendications précédentes, dans lequel la ou les molécules polymérisables sont choisies parmi celles de formule **(IIa)** ci-dessous :

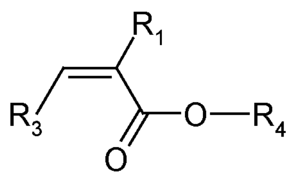

et également leurs sels d'acide ou de base, organique ou minéral(e), leurs isomères optiques, leurs isomères géométriques et leurs formes tautomères, et leurs solvates tels que les hydrates ; dans laquelle formule **(IIa), $R_1$,** **$R_3$**, et **$R_4$** étant tels que définis dans les revendications 5 à 9 ; préférablement la ou les molécules polymérisables ii) sont choisies parmi un acide (($C_1$-$C_4$)alkyl)acrylique tel que l'acide méthacrylique, l'acide acrylique ou l'acide itaconique.

**11.** Procédé selon l'une quelconque des revendications précédentes, dans lequel la ou les molécules polymérisables sont choisies parmi celles de formule **(IIb)** ci-dessous :

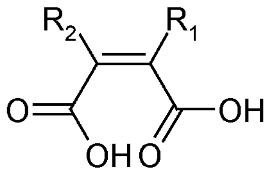

(IIb)

et également leurs sels d'acide ou de base, organique ou minéral(e), leurs isomères optiques, leurs isomères géométriques et leurs formes tautomères, et leurs solvates tels que les hydrates ;
dans laquelle formule **(IIb)** , **$R_1$,** et **$R_2$**, sont tels que définis dans les revendications 5 à 10, en particulier choisis parmi hydrogène et ($C_1$-$C_4$) alkyle tel que méthyle, préférablement hydrogène.

**12.** Procédé selon l'une quelconque des revendications précédentes, dans lequel la ou les molécules polymérisables sont choisies parmi celles de formule **(IIc)** ci-dessous :

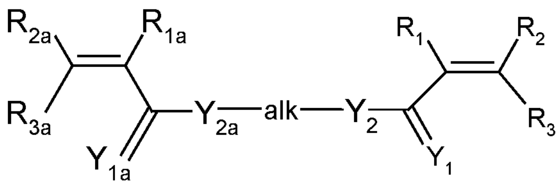

(IIc)

et également leurs sels d'acide ou de base, organique ou minéral(e), leurs isomères optiques, leurs isomères géométriques et leurs formes tautomères, et leurs solvates tels que les hydrates ;
dans laquelle formule **(IIc), $R_1$, $R_2$,** $R_3$, $Y_1$, $Y_2$, alk, $Y_{2a}$, $Y_{1a}$, $R_{1a}$, $R_{2a}$ et $R_{3a}$ sont tels que définis dans les revendications 5 à 11, en particulier tels que $R_1$ = $R_{1a}$ et $R_2$ = $R_{2a}$ et $R_3$ = $R_{3a}$ et $Y_1$= $Y_{1a}$ et $Y_2$=$Y_{2a}$ ; préférentiellement choisies parmi les composés (a) à (d) ci-dessous :

| | |
|---|---|
| 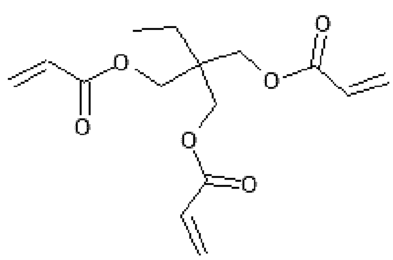 | 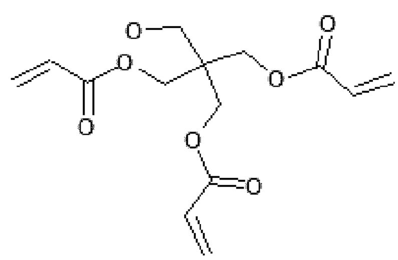 |
| **(a)** | **(b)** |

(suite)

| | |
|---|---|
| 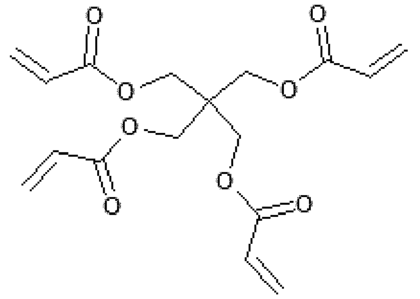 | 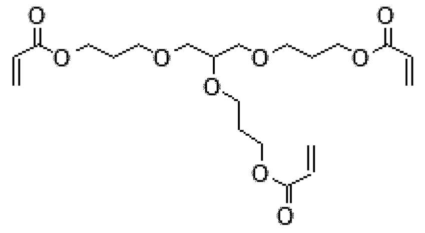 |
| **(c)** | **(d)** |

**13.** Procédé selon l'une quelconque des revendications précédentes, dans lequel la ou les molécules polymérisables se trouvent dans une quantité comprise inclusivement entre 0,01 % et 50 % en poids par rapport au poids total des compositions (A) ou (B), en particulier entre 0,1 % et 40 %, plus particulièrement entre 1 % et 30 %, préférentiellement entre 2 % et 20 %, plus préférentiellement entre 5 % et 10 % en poids par rapport au poids total des compositions (A) ou (B).

**14.** Procédé selon l'une quelconque des revendications précédentes, dans lequel la composition (A) ou (B) comprend une ou plusieurs amines différentes i) des riboflavines de formule **(I)**, et différentes ii) des molécules polymérisables ; lesdites amines pouvant être aromatiques ou non aromatiques, saturées ou insaturées, oligomériques ou polymériques, et naturelles ou synthétiques, préférablement l'amine ou les amines étant des acides aminés, choisis parmi les composés de formule **(III)** ci-dessous :

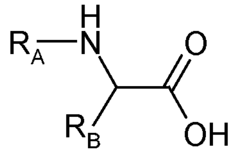

**(III)**

et également leurs sels d'acide ou de base, organique ou minéral(e), leurs isomères optiques, leurs isomères géométriques et leurs formes tautomères, et leurs solvates tels que les hydrates ;

- **$R_A$** représente un atome d'hydrogène, un groupe $(C_1\text{-}C_4)$alkyle ou $(C_1\text{-}C_4)$alkylcarbonyle ou alors forme ensemble avec le radical $R_B$ et l'atome d'azote et de carbone qui les portent respectivement un hétérocycle monocyclique ou bicyclique, comprenant de 5 à 10 chainons telle que pyrolidinyle ; préférablement $R_A$ représente un atome d'hydrogène ;
- **$R_B$** représente un atome d'hydrogène ou un groupe $(C_1\text{-}C_6)$alkyle éventuellement substitué par un ou plusieurs groupes choisis parmi i) -Z-C(Z')-Z"-$R_c$, Z, Z', et Z", qui peuvent être identiques ou différents, représentant un atome d'oxygène ou de soufre, et N($R_D$), $R_C$ et $R_D$, qui peuvent être identiques ou différents, représentant un atome d'hydrogène ou un groupe $(C_1\text{-}C_4)$ alkyle ; préférablement Z, Z' et Z" représentent un groupe N($R_D$) tel que NH ; ii) (hétéro)aryle tel que imidazolyle, indolyle, phényle, eventuellement substitué notamment par un groupe hydroxyle ; iii) (di) $(C_1\text{-}C_4)$ (alkyl) amino, iv) -C(Z')-Z"-$R_C$ avec Z', Z" et $R_C$ tels que définis précédemment, en particulier Z' représente un atome d'oxygène, Z" représente un atome d'oxygène ou un groupe N($R_D$) tel que NH, et $R_c$ représente un atome d'hydrogène ; v) - Z'''-$R_C$, Z''' représentant un atome d'oxygène, de soufre ou de sélénium ou un groupe NH et $R_C$ étant tel que défini précédemment ; en particulier RB représente un groupe $(C_1\text{-}C_6)$alkyle éventuellement substitué par i) -Z-C(Z')-Z"-$R_C$, Z, Z', et Z", qui peuvent être identiques ou différents, représentant un atome d'oxygène ou de soufre, et N ($R_D$), Rc et $R_D$, qui peuvent être identiques ou différents, représentant un atome d'hydrogène ou un groupe $(C_1\text{-}C_4)$alkyle ; préférablement Z, Z' et Z" représentent un groupe N($R_D$) tel que NH ;

ou $R_A$ et $R_B$ forment ensemble avec l'atome d'azote qui porte $R_A$ et avec l'atome de carbone qui porte $R_B$ un hétérocycle saturé à 5 ou 6 chainons, préférablement 5 chainons tel qu'un cycle pyrrolidine.

**15.** Procédé selon l'une quelconque des revendications précédentes, dans lequel l'étape 2) d'apport énergétique consiste à exposer les matières kératiniques à un rayonnement lumineux artificiel à une longueur d'onde comprise entre 360 et 600 nm, préférablement entre 375 et 550 nm, plus préférentiellement entre 400 et 480 nm ; préférablement, le rayonnement lumineux artificiel est généré à l'aide d'un dispositif choisi parmi les lampes d'arc telles que les lampes à xénon et les lampes à mercure, les lampes fluorescentes, les lampes à incandescence telles que les lampes halogènes, les diodes luminescentes (LED), les diodes électroluminescentes organiques (OLED) et les lasers.

**16.** Procédé pour la préparation de la composition (B'), qui comprend l'étape de mélange i) d'un ou de plusieurs composés de formule **(I)** tels que définis dans une quelconque des revendications 1 à 7, ii) d'une ou de plusieurs molécules polymérisables choisies parmi celles de formule **(II), (IIa), (IIb)** et **(IIc)** telles que définies dans une quelconque des revendications 1 et 9 à 13 et éventuellement iii) une ou plusieurs amines et/ou un ou plusieurs alcools différents i) des composés de formule (**I**), et différents ii) des molécules polymérisables, préférablement une ou plusieurs amines, préférablement choisies parmi les acides aminés tels que définis dans la revendication 14 ; suivie d'une étape d'exposition dudit mélange à un rayonnement lumineux artificiel ou naturel tel que défini dans la revendication 1 ou 15, en particulier pendant au moins 2 minutes, préférablement pendant une durée de 2 minutes jusqu'à 10 heures, plus préférentiellement entre 15 minutes et 5 heures, telle que 3 heures.

**17.** Composition (B') obtenue par le procédé selon la revendication précédente.

**18.** Dispositif à plusieurs compartiments :

- comprenant :

  * soit dans un compartiment l'ingrédient i) un ou plusieurs composés de formule **(I)** tels que définis dans une quelconque des revendications 1 à 7, et dans un autre compartiment l'ingrédient ii) une ou plusieurs molécules polymérisables choisies parmi celles de formule **(II), (IIa), (IIb)** ou **(IIc)** telles que définies dans l'une quelconque des revendications 1 et 9 à 13,
  * soit dans un compartiment les ingrédients i) un ou plusieurs composés de formule **(I)** tels que définis dans une quelconque des revendications 1 à 7, et ii) une ou plusieurs molécules polymérisables choisies parmi celles de formule **(II), (IIa), (IIb)** ou **(IIc)** telles que définies dans l'une quelconque des revendications 1 et 9 à 13, i) et ii) étant ensemble, et

- dans un autre compartiment un dispositif emetteur de lumière artificielle tel que défini dans la revendication 15.

**19.** Utilisation cosmétique i) d'un ou de plusieurs dérivés de flavine choisis parmi les composés de formule **(I)** tels que définis dans l'une quelconque des revendications 1 à 7, et ii) d'une ou plusieurs molécules polymérisables choisies parmi celles de formule **(II), (IIa), (IIb)** ou **(IIc)** telles que définies dans l'une quelconque des revendications 1 et 9 à 13, et d'un rayonnement lumineux artificiel ou naturel tel que défini dans la revendication 1 ou 15 ; en particulier pour le soin et/ou la réparation de matières kératiniques, notamment de matières kératiniques humaines telles que les cheveux, préférablement pour la réparation des cheveux.

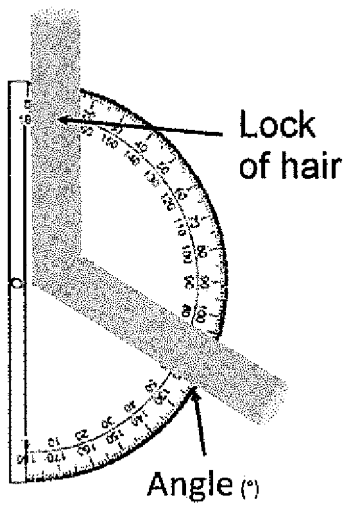


Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010083039 A
- WO 2016126121 A


### Non-patent literature cited in the description


- **KIM, S.-H. ; CHU, C.-C.** *J. Biomed. Mater. Res. B. Appl. Biomater.,* 2009, vol. 91 (1), 390-400
- **PIYUSH GUPTA ; KAVITA VERMANI ; SANJAY GARG.** *Drug. Discovery Today,* 2002, vol. 7 (10
- *CHEMICAL ABSTRACTS,* 752-56-7
- *CHEMICAL ABSTRACTS,* 4074-58-2
- Biosynthesis of Vitamin B2 (Riboflavin). *Annual Review of Nutrition,* July 2000, vol. 20, 153-167, https://doi.org/10.1146/annurev.nutr.20.1.153